# EUROPEAN PATENT APPLICATION

(11) **EP 1 647 600 A2**
(43) Date of publication of application: **19.04.2006**
(21) Application number: 05255780.8
(22) Date of filing: 19.09.2005
(51) Int. Cl.: C12Q 1/68

(54) **Methods for identifying biological samples by addition of nucleic acid bar-code tags**

(30) Priority: 17.09.2004 US 610668 P
(71) Applicant: Affymetrix, Inc. (A US Entity), Santa Clara, California 95051 (US)
(72) Inventor: Christians, Frederick C., Los Altos Hills, CA 94022 (US); Mei, Rui, Santa Clara, CA 95051 (US)
(74) Representative: Bizley, Richard Edward

(57) **Abstract**

The present invention provides methods for marking nucleic acid samples with detectable barcodes by adding different combinations of marker molecules to each sample. Each sample may be marked with a different combination of two or more marker molecules each carrying a different tag nucleic acid sequences. The tag nucleic acid sequences may be random sequences that are not naturally occurring in the nucleic acid sample and do not cross hybridize to sequences naturally occurring in the nucleic acid sample. Methods of detecting the barcode in parallel with methods of genetic analysis of the sample are disclosed. Kits containing marker molecules suitable for generating barcoded samples by mixing different combinations of marker molecules are also disclosed.

SEQUENCE LISTING

<110>  Affymetrix, Inc.

<120>  Methods for Identifying Biological Samples

<130>  AFMX-PEP-045

<150>  60/610,668
<151>  2004-09-17

<160>  88

<170>  PatentIn version 3.2

<210>  1
<211>  40
<212>  DNA
<213>  Artificial sequence

<220>
<223>  Synthetic oligonucleotide

<400>  1
tgacattatt ggttcggagc tgactatctt ggtgtcgcgc                                40


<210>  2
<211>  40
<212>  DNA
<213>  Artificial sequence

<220>
<223>  Synthetic oligonucleotide

<400>  2
taccgctgtg tgtatgtcgc tgccgctctt tatgctacgg                                40


<210>  3
<211>  40
<212>  DNA
<213>  Artificial sequence

<220>
<223>  Synthetic oligonucleotide

<400>  3
ctgcgctcta gttacatcat gctctcatta gttagtaggc                                40


<210>  4
<211>  40
<212>  DNA
<213>  Artificial sequence

<220>
<223>  Synthetic oligonucleotide

<400>  4
gctcgttatg tatgtagacg gttgtatcaa tgtgtgcgac                                40


<210>  5
<211>  40
<212>  DNA
<213>  Artificial sequence

<220>
<223>  Synthetic oligonucleotide

<400>  5
gtcgaatatc tctgtgtgag ggtatcttca tctgtggagc                                40


<210>  6
<211>  40
<212>  DNA
<213>  Artificial sequence

<220>
<223>  Synthetic oligonucleotide

<400>  6
gagtctctgt actgtgagct tagtccagtt gattagtggc                                40


<210>  7
<211>  40
<212>  DNA
<213>  Artificial sequence

<220>
<223>  Synthetic oligonucleotide

<400>  7
tcgcgtcatt tatcgaatcg tgaggtgctt tgtactaacg                                40


<210>  8
<211>  40
<212>  DNA
<213>  Artificial sequence

<220>
<223>  Synthetic oligonucleotide

<400>  8
gagttatatc tgtgctacgg gcgtaatctc tgtctagctc                                40


<210>  9
<211>  40
<212>  DNA
<213>  Artificial sequence

<220>
<223>  Synthetic oligonucleotide

<400>  9
gcgtgtactg tatctcgtgc gcgtgtaatc tgtctctccg                                40


<210>  10
<211>  40
<212>  DNA
<213>  Artificial sequence

<220>
<223>  Synthetic oligonucleotide

<400>  10
gatctgatta cgtctctcgc cgcatatcta cgtctctagg                                40


<210>  11

<211>  40
<212>  DNA
<213>  Artificial sequence

<220>
<223>  Synthetic oligonucleotide

<400>  11
agtgatcgca ccttatctcg gtctgacttg agttacatgg                                40


<210>  12
<211>  40
<212>  DNA
<213>  Artificial sequence

<220>
<223>  Synthetic oligonucleotide

<400>  12
gtgctacttg atctacatgg gcttactatt catactgccg                                40


<210>  13
<211>  40
<212>  DNA
<213>  Artificial sequence

<220>
<223>  Synthetic oligonucleotide

<400>  13
gctctacgtt catatcatgg cctatcgctc tatatctggg                                40


<210>  14
<211>  40
<212>  DNA
<213>  Artificial sequence

<220>
<223>  Synthetic oligonucleotide

<400>  14
cgtatcgtgc tacctgctag gagtgtcctg tacgttagcc                                40


<210>  15
<211>  40
<212>  DNA
<213>  Artificial sequence

<220>
<223>  Synthetic oligonucleotide

<400>  15
atatgtacga agctagtggc tatagttatt gacgctgcgg                                40


<210>  16
<211>  40
<212>  DNA
<213>  Artificial sequence

<220>
<223>  Synthetic oligonucleotide

<400>  16

ttatgctttc tacgccgggc tgtctatgtt tacagcgggc                                40

<210>  17
<211>  40
<212>  DNA
<213>  Artificial sequence

<220>
<223>  Synthetic oligonucleotide

<400>  17
tgcttatctt tacaccggcg tgtaatgatt tccacgctgg                                40

<210>  18
<211>  40
<212>  DNA
<213>  Artificial sequence

<220>
<223>  Synthetic oligonucleotide

<400>  18
tctaatcatt tacgacgcgg tgatactatt gtcgtcgggc                                40

<210>  19
<211>  40
<212>  DNA
<213>  Artificial sequence

<220>
<223>  Synthetic oligonucleotide

<400>  19
tatacttatt gaggtcgcgg tctgacttta gatgtcgggc                                40

<210>  20
<211>  40
<212>  DNA
<213>  Artificial sequence

<220>
<223>  Synthetic oligonucleotide

<400>  20
catgactatt aacctagcgg ggtcttgcca acgtctgtga                                40

<210>  21
<211>  20
<212>  DNA
<213>  Artificial sequence

<220>
<223>  Synthetic oligonucleotide

<400>  21
aacgccaggg ttttcccagt                                                      20

<210>  22
<211>  21
<212>  DNA
<213>  Artificial sequence

<220>
<223>  Synthetic oligonucleotide

<400>  22
caggaaacag ctatgaccat g                                                21


<210>  23
<211>  40
<212>  DNA
<213>  Artificial sequence

<220>
<223>  Synthetic oligonucleotide

<400>  23
cgttatcaac ctgcgtccga gcagtaattt caatcgcgtg                            40


<210>  24
<211>  40
<212>  DNA
<213>  Artificial sequence

<220>
<223>  Synthetic oligonucleotide

<400>  24
ccttatccac ctgagtgagt gaggtagttt ccacgctatg                            40


<210>  25
<211>  40
<212>  DNA
<213>  Artificial sequence

<220>
<223>  Synthetic oligonucleotide

<400>  25
cgtgttcaaa gcgcgtacct gcgaatagtt ccacgtctgg                            40


<210>  26
<211>  40
<212>  DNA
<213>  Artificial sequence

<220>
<223>  Synthetic oligonucleotide

<400>  26
cgccttagac acgtcgtaga ctactgagtt acagtctgac                            40


<210>  27
<211>  40
<212>  DNA
<213>  Artificial sequence

<220>
<223>  Synthetic oligonucleotide

<400>  27
cggtcttata ccactgtaga gcgatgactg ataatacacg                            40


<210>  28

<211>  40
<212>  DNA
<213>  Artificial sequence

<220>
<223>  Synthetic oligonucleotide

<400>  28
cgctggataa tcacctgagg cgatgcactg tcatacgata                              40


<210>  29
<211>  40
<212>  DNA
<213>  Artificial sequence

<220>
<223>  Synthetic oligonucleotide

<400>  29
gaggatgtta ccactctgac cgacacgatg gtgcaactgt                              40


<210>  30
<211>  40
<212>  DNA
<213>  Artificial sequence

<220>
<223>  Synthetic oligonucleotide

<400>  30
gataatgtta ccatacgcgc cgatgtcatc tggctacggt                              40


<210>  31
<211>  40
<212>  DNA
<213>  Artificial sequence

<220>
<223>  Synthetic oligonucleotide

<400>  31
ttagtatgtt tcacacggcg catatagctc tagtatccgc                              40


<210>  32
<211>  40
<212>  DNA
<213>  Artificial sequence

<220>
<223>  Synthetic oligonucleotide

<400>  32
gtactagata ctcacatcgg cagaacctga tatgctcgcg                              40


<210>  33
<211>  40
<212>  DNA
<213>  Artificial sequence

<220>
<223>  Synthetic oligonucleotide

<400>  33

gttcttcatt ctacgcacgg atgaacatct atcgctcgct 40

<210> 34
<211> 40
<212> DNA
<213> Artificial sequence

<220>
<223> Synthetic oligonucleotide

<400> 34
ctacactatt ctacacctcg catgagactg tactaagcgt 40

<210> 35
<211> 40
<212> DNA
<213> Artificial sequence

<220>
<223> Synthetic oligonucleotide

<400> 35
ttgaatggtt tcaatcgcgg atatgactgg aatagccgtg 40

<210> 36
<211> 40
<212> DNA
<213> Artificial sequence

<220>
<223> Synthetic oligonucleotide

<400> 36
agaagctata ctatcgcacc agcagaactc tatacacctg 40

<210> 37
<211> 40
<212> DNA
<213> Artificial sequence

<220>
<223> Synthetic oligonucleotide

<400> 37
ctgcaattat ctactctgcg agtacaatgc catacgctct 40

<210> 38
<211> 40
<212> DNA
<213> Artificial sequence

<220>
<223> Synthetic oligonucleotide

<400> 38
cgcacttcaa caatcgtgta agtagacgtg catagcagtt 40

<210> 39
<211> 40
<212> DNA
<213> Artificial sequence

<220>
<223>  Synthetic oligonucleotide

<400>  39
cggctatgta cgacgtgcta cgctgacctg tctaacgtat                              40


<210>  40
<211>  40
<212>  DNA
<213>  Artificial sequence

<220>
<223>  Synthetic oligonucleotide

<400>  40
gcggctaatt cgacgctgta gcccgcgctt cataagtgta                              40


<210>  41
<211>  40
<212>  DNA
<213>  Artificial sequence

<220>
<223>  Synthetic oligonucleotide

<400>  41
cacacccgtg cataaggtat tcccgcgatg accgagaatt                              40


<210>  42
<211>  40
<212>  DNA
<213>  Artificial sequence

<220>
<223>  Synthetic oligonucleotide

<400>  42
aggccgctgg cacagtatat tcgcggcggt cagacaatat                              40


<210>  43
<211>  4988
<212>  DNA
<213>  Artificial sequence

<220>
<223>  Synthetic oligonucleotide

<400>  43
ccattcaggc tgcgcaactg ttgggaaggg cgatcggtgc gggcctcttc gctattacgc      60

cagctggcga aagggggatg tgctgcaagg cgattaagtt gggtaacgcc agggttttcc     120

cagtcacgac gttgtaaaac gacggccagt gaattgaatt taggtgacac tatagaagag     180

ctatgacgtc gcatgcaatt aaccctcact aaagggacgc gtacgtaagc ttggatcctc     240

tagagcggcc gcttatttgt agagctcatc catgccatgt gtaatcccag cagcagttac     300

aaactcaaga aggaccatgt ggtcacgctt ttcgttggga tctttcgaaa gggcagattg     360

tgtcgacagg taatggttgt ctggtaaaag gacagggcca tcgccaattg gagtattttg     420

ttgataatgg tctgctagtt gaacggatcc atcttcaatg ttgtggcgaa ttttgaagtt     480

agctttgatt ccattctttt gtttgtctgc cgtgatgtat acattgtgtg agttatagtt 540
gtactcgagt ttgtgtccga gaatgtttcc atcttcttta aaatcaatac cttttaactc 600
gatacgatta acaagggtat caccttcaaa cttgacttca gcacgcgtct tgtagttccc 660
gtcatctttg aaagatatag tgcgttcctg tacataacct tcgggcatgg cactcttgaa 720
aaagtcatgc cgtttcatat gatccggata acgggaaaag cattgaacac cataagagaa 780
agtagtgaca agtgttggcc atggaacagg tagttttcca gtagtgcaaa taaatttaag 840
ggtaagcttt ccgtatgtag catcaccttc accctctcca ctgacagaaa atttgtgccc 900
attaacatca ccatctaatt caacaagaat tgggacaact ccagtgaaaa gttcttctcc 960
tttgctagca gacccacgag gaacaaggcc gctgctgtga tgatggtggt gatgattaat 1020
attattattg ctaatgttag caatcatatg tatattcctt ctgcggccgc gtcgaccccc 1080
gggaattccg gaaaaaaaaa aaaaaaaaaa aactgcagtc tagaaagctt ctgcagggcg 1140
cgccatttaa attgcaggcg taccagcttt ccctatagtg agtcgtatta gagcttggcg 1200
taatcatggt catagctgtt tcctgtgtga aattgttatc cgctcacaat tccacacaac 1260
atacgagccg gaagcataaa gtgtaaagcc tggggtgcct aatgagtgag ctaactcaca 1320
ttaattgcgt tgcgctcact gcccgctttc cagtcgggaa acctgtcgtg ccagctgcat 1380
taatgaatcg gccaacgcgc ggggagaggc ggtttgcgta ttgggcgcca gggtggtttt 1440
tcttttcacc agtgagacgg gcaacagctg attgcccttc accgcctggc cctgagagag 1500
ttgcagcaag cggtccacgc tggtttgccc cagcaggcga aaatcctgtt tgatggtggt 1560
taacggcggg atataacatg agctgtcttc ggtatcgtcg tatcccacta ccgagatatc 1620
cgcaccaacg cgcagcccgg actcggtaat ggcgcgcatt gcgcccagcg ccatctgatc 1680
gttggcaacc agcatcgcag tgggaacgat gccctcattc agcatttgca tggtttgttg 1740
aaaaccggac atggcactcc agtcgccttc ccgttccgct atcggctgaa tttgattgcg 1800
agtgagatat ttatgccagc cagccagacg cagacgcgcc gagacagaac ttaatgggcc 1860
cgctaacagc gcgatttgct ggtgacccaa tgcgaccaga tgctccacgc ccagtcgcgt 1920
accgtcttca tgggagaaaa taatactgtt gatgggtgtc tggtcagaga catcaagaaa 1980
taacgccgga acattagtgc aggcagcttc cacagcaatg gcatcctggt catccagcgg 2040
atagttaatg atcagcccac tgacccgttg cgcgagaaga ttgtgcaccg ccgctttaca 2100
ggcttcgacg ccgcttcgtt ctaccatcga caccaccacg ctggcaccca gttgatcggc 2160
gcgagattta atcgccgcga caatttgcga cggcgcgtgc agggccagac tggaggtggc 2220
aacgccaatc agcaacgact gtttgcccgc cagttgttgt gccacgcggt tgggaatgta 2280
attcagctcc gccatcgccg cttccacttt ttcccgcgtt ttcgcagaaa cgtggctggc 2340
ctggttcacc acgcgggaaa cggtctgata agagacaccg gcatactctg cgacatcgta 2400
taacgttact ggtttcacat tcaccaccct gaattgactc tcttccgggc gctatcatgc 2460
cataccgcga aaggttttgc gccattcgat ggtgtcaacg taaatgccgc ttcgccttcg 2520

cgcgcgaatt gcaagctctg cattaatgaa tcggccaacg cgcggggaga ggcggtttgc 2580
gtattgggcg ctcttccgct tcctcgctca ctgactcgct gcgctcggtc gttcggctgc 2640
ggcgagcggt atcagctcac tcaaaggcgg taatacggtt atccacagaa tcaggggata 2700
acgcaggaaa gaacatgtga gcaaaaggcc agcaaaaggc caggaaccgt aaaaaggccg 2760
cgttgctggc gtttttccat aggctccgcc cccctgacga gcatcacaaa aatcgacgct 2820
caagtcagag gtggcgaaac ccgacaggac tataaagata ccaggcgttt ccccctggaa 2880
gctccctcgt gcgctctcct gttccgaccc tgccgcttac cggatacctg tccgcctttc 2940
tcccttcggg aagcgtggcg ctttctcaat gctcacgctg taggtatctc agttcggtgt 3000
aggtcgttcg ctccaagctg ggctgtgtgc acgaaccccc cgttcagccc gaccgctgcg 3060
ccttatccgg taactatcgt cttgagtcca acccggtaag acacgactta tcgccactgg 3120
cagcagccac tggtaacagg attagcagag cgaggtatgt aggcggtgct acagagttct 3180
tgaagtggtg gcctaactac ggctacacta gaaggacagt atttggtatc tgcgctctgc 3240
tgaagccagt taccttcgga aaaagagttg gtagctcttg atccggcaaa caaaccaccg 3300
ctggtagcgg tggttttttt gtttgcaagc agcagattac gcgcagaaaa aaaggatctc 3360
aagaagatcc tttgatcttt tctacggggt ctgacgctca gtggaacgaa aactcacgtt 3420
aagggatttt ggtcatgaga ttatcaaaaa ggatcttcac ctagatcctt ttaaattaaa 3480
aatgaagttt taaatcaatc taaagtatat atgagtaaac ttggtctgac agttaccaat 3540
gcttaatcag tgaggcacct atctcagcga tctgtctatt tcgttcatcc atagttgcct 3600
gactccccgt cgtgtagata actacgatac gggagggctt accatctggc cccagtgctg 3660
caatgatacc gcgagaccca cgctcaccgg ctccagattt atcagcaata aaccagccag 3720
ccggaagggc cgagcgcaga agtggtcctg caactttatc cgcctccatc cagtctatta 3780
attgttgccg ggaagctaga gtaagtagtt cgccagttaa tagtttgcgc aacgttgttg 3840
ccattgctac aggcatcgtg gtgtcacgct cgtcgtttgg tatggcttca ttcagctccg 3900
gttcccaacg atcaaggcga gttacatgat cccccatgtt gtgcaaaaaa gcggttagct 3960
ccttcggtcc tccgatcgtt gtcagaagta agttggccgc agtgttatca ctcatggtta 4020
tggcagcact gcataattct cttactgtca tgccatccgt aagatgcttt tctgtgactg 4080
gtgagtactc aaccaagtca ttctgagaat agtgtatgcg gcgaccgagt tgctcttgcc 4140
cggcgtcaat acgggataat accgcgccac atagcagaac tttaaaagtg ctcatcattg 4200
gaaaacgttc ttcggggcga aaactctcaa ggatcttacc gctgttgaga tccagttcga 4260
tgtaacccac tcgtgcaccc aactgatctt cagcatcttt tactttcacc agcgtttctg 4320
ggtgagcaaa aacaggaagg caaaatgccg caaaaaaggg aataagggcg acacggaaat 4380
gttgaatact catactcttc ctttttcaat attattgaag catttatcag ggttattgtc 4440
tcatgagcgg atacatattt gaatgtattt agaaaaataa acaaataggg gttccgcgca 4500
catttccccg aaaagtgcca cctgaaattg taaacgttaa tattttgtta aaattcgcgt 4560

taaatttttg ttaaatcagc tcatttttta accaataggc cgaaatcggc aaaatccctt 4620
ataaatcaaa agaatagacc gagatagggt tgagtgttgt tccagtttgg aacaagagtc 4680
cactattaaa gaacgtggac tccaacgtca aagggcgaaa aaccgtctat cagggcgatg 4740
gcccactacg tgaaccatca ccctaatcaa gttttttggg gtcgaggtgc cgtaaagcac 4800
taaatcggaa ccctaaaggg agcccccgat ttagagcttg acggggaaag ccggcgaacg 4860
tggcgagaaa ggaagggaag aaagcgaaag gagcgggcgc tagggcgctg gcaagtgtag 4920
cggtcacgct gcgcgtaacc accacacccg ccgcgcttaa tgcgccgcta cagggcgcgt 4980
cccattcg 4988

<210> 44
<211> 4620
<212> DNA
<213> Artificial sequence

<220>
<223> Synthetic oligonucleotide

<400> 44
ccattcaggc tgcgcaactg ttgggaaggg cgatcggtgc gggcctcttc gctattacgc 60
cagctggcga aagggggatg tgctgcaagg cgattaagtt gggtaacgcc agggttttcc 120
cagtcacgac gttgtaaaac gacggccagt gaattgaatt taggtgacac tatagaagag 180
ctatgacgtc gcatgcaatt aaccctcact aaagggacgc gtacgtaagc ttggatcctc 240
tagagcggcc gcttatttgt agagctcatc catgccatgt gtaatcccag cagcagttac 300
aaactcaaga aggaccatgt ggtcacgctt ttcgttggga tctttcgaaa gggcagattg 360
tgtcgacagg taatggttgt ctggtaaaag gacagggcca tcgccaattg gagtattttg 420
ttgataatgg tctgctagtt gaacggatcc atcttcaatg ttgtggcgaa ttttgaagtt 480
agctttgatt ccattctttt gtttgtctgc cgtgatgtat acattgtgtg agttatagtt 540
gtactcgaga ctagttgaca ttattggttc ggagctgact atcttggtgt cgcgcgctag 600
cagacccacg aggaacaagg ccgctgctgt gatgatggtg gtgatgatta atattattat 660
tgctaatgtt agcaatcata tgtatattcc ttctgcggcc gcgtcgaccc ccgggaattc 720
cggaaaaaaa aaaaaaaaaa aaaactgcag tctagaaagc ttctgcaggg cgcgccattt 780
aaattgcagg cgtaccagct ttccctatag tgagtcgtat tagagcttgg cgtaatcatg 840
gtcatagctg tttcctgtgt gaaattgtta tccgctcaca attccacaca acatacgagc 900
cggaagcata aagtgtaaag cctggggtgc ctaatgagtg agctaactca cattaattgc 960
gttgcgctca ctgcccgctt tccagtcggg aaacctgtcg tgccagctgc attaatgaat 1020
cggccaacgc gcggggagag gcggtttgcg tattgggcgc cagggtggtt tttcttttca 1080
ccagtgagac gggcaacagc tgattgccct tcaccgcctg gccctgagag agttgcagca 1140
agcggtccac gctggtttgc cccagcaggc gaaaatcctg tttgatggtg gttaacggcg 1200
ggatataaca tgagctgtct tcggtatcgt cgtatcccac taccgagata tccgcaccaa 1260

cgcgcagccc ggactcggta atggcgcgca ttgcgcccag cgccatctga tcgttggcaa 1320
ccagcatcgc agtgggaacg atgccctcat tcagcatttg catggtttgt tgaaaaccgg 1380
acatggcact ccagtcgcct tcccgttccg ctatcggctg aatttgattg cgagtgagat 1440
atttatgcca gccagccaga cgcagacgcg ccgagacaga acttaatggg cccgctaaca 1500
gcgcgatttg ctggtgaccc aatgcgacca gatgctccac gcccagtcgc gtaccgtctt 1560
catgggagaa aataatactg ttgatgggtg tctggtcaga gacatcaaga aataacgccg 1620
gaacattagt gcaggcagct tccacagcaa tggcatcctg gtcatccagc ggatagttaa 1680
tgatcagccc actgacccgt tgcgcgagaa gattgtgcac cgccgcttta caggcttcga 1740
cgccgcttcg ttctaccatc gacaccacca cgctggcacc cagttgatcg gcgcgagatt 1800
taatcgccgc gacaatttgc gacggcgcgt gcagggccag actggaggtg gcaacgccaa 1860
tcagcaacga ctgtttgccc gccagttgtt gtgccacgcg gttgggaatg taattcagct 1920
ccgccatcgc cgcttccact ttttcccgcg ttttcgcaga aacgtggctg gcctggttca 1980
ccacgcggga aacggtctga taagagacac cggcatactc tgcgacatcg tataacgtta 2040
ctggtttcac attcaccacc ctgaattgac tctcttccgg gcgctatcat gccataccgc 2100
gaaaggtttt gcgccattcg atggtgtcaa cgtaaatgcc gcttcgcctt cgcgcgcgaa 2160
ttgcaagctc tgcattaatg aatcggccaa cgcgcgggga gaggcggttt gcgtattggg 2220
cgctcttccg cttcctcgct cactgactcg ctgcgctcgg tcgttcggct gcggcgagcg 2280
gtatcagctc actcaaaggc ggtaatacgg ttatccacag aatcagggga taacgcagga 2340
aagaacatgt gagcaaaagg ccagcaaaag gccaggaacc gtaaaaaggc cgcgttgctg 2400
gcgtttttcc ataggctccg cccccctgac gagcatcaca aaaatcgacg ctcaagtcag 2460
aggtggcgaa acccgacagg actataaaga taccaggcgt ttccccctgg aagctccctc 2520
gtgcgctctc ctgttccgac cctgccgctt accggatacc tgtccgcctt tctcccttcg 2580
ggaagcgtgg cgctttctca atgctcacgc tgtaggtatc tcagttcggt gtaggtcgtt 2640
cgctccaagc tgggctgtgt gcacgaaccc cccgttcagc ccgaccgctg cgccttatcc 2700
ggtaactatc gtcttgagtc caacccggta agacacgact tatcgccact ggcagcagcc 2760
actggtaaca ggattagcag agcgaggtat gtaggcggtg ctacagagtt cttgaagtgg 2820
tggcctaact acggctacac tagaaggaca gtatttggta tctgcgctct gctgaagcca 2880
gttaccttcg gaaaaagagt tggtagctct tgatccggca aacaaaccac cgctggtagc 2940
ggtggttttt ttgtttgcaa gcagcagatt acgcgcagaa aaaaaggatc tcaagaagat 3000
cctttgatct tttctacggg gtctgacgct cagtggaacg aaaactcacg ttaagggatt 3060
ttggtcatga gattatcaaa aaggatcttc acctagatcc ttttaaatta aaaatgaagt 3120
tttaaatcaa tctaaagtat atatgagtaa acttggtctg acagttacca atgcttaatc 3180
agtgaggcac ctatctcagc gatctgtcta tttcgttcat ccatagttgc ctgactcccc 3240
gtcgtgtaga taactacgat acgggagggc ttaccatctg gccccagtgc tgcaatgata 3300

ccgcgagacc cacgctcacc ggctccagat ttatcagcaa taaaccagcc agccggaagg 3360
gccgagcgca gaagtggtcc tgcaacttta tccgcctcca tccagtctat taattgttgc 3420
cgggaagcta gagtaagtag ttcgccagtt aatagtttgc gcaacgttgt tgccattgct 3480
acaggcatcg tggtgtcacg ctcgtcgttt ggtatggctt cattcagctc cggttcccaa 3540
cgatcaaggc gagttacatg atcccccatg ttgtgcaaaa aagcggttag ctccttcggt 3600
cctccgatcg ttgtcagaag taagttggcc gcagtgttat cactcatggt tatggcagca 3660
ctgcataatt ctcttactgt catgccatcc gtaagatgct tttctgtgac tggtgagtac 3720
tcaaccaagt cattctgaga atagtgtatg cggcgaccga gttgctcttg cccggcgtca 3780
atacgggata ataccgcgcc acatagcaga actttaaaag tgctcatcat tggaaaacgt 3840
tcttcggggc gaaaactctc aaggatctta ccgctgttga gatccagttc gatgtaaccc 3900
actcgtgcac ccaactgatc ttcagcatct tttactttca ccagcgtttc tgggtgagca 3960
aaaacaggaa ggcaaaatgc cgcaaaaaag ggaataaggg cgacacggaa atgttgaata 4020
ctcatactct tcctttttca atattattga agcatttatc agggttattg tctcatgagc 4080
ggatacatat ttgaatgtat ttagaaaaat aaacaaatag gggttccgcg cacatttccc 4140
cgaaaagtgc cacctgaaat tgtaaacgtt aatattttgt taaaattcgc gttaaatttt 4200
tgttaaatca gctcattttt taaccaatag gccgaaatcg gcaaaatccc ttataaatca 4260
aaagaataga ccgagatagg gttgagtgtt gttccagttt ggaacaagag tccactatta 4320
aagaacgtgg actccaacgt caaagggcga aaaaccgtct atcagggcga tggcccacta 4380
cgtgaaccat caccctaatc aagttttttg gggtcgaggt gccgtaaagc actaaatcgg 4440
aaccctaaag ggagcccccg atttagagct tgacggggaa agccggcgaa cgtggcgaga 4500
aaggaaggga agaaagcgaa aggagcgggc gctagggcgc tggcaagtgt agcggtcacg 4560
ctgcgcgtaa ccaccacacc cgccgcgctt aatgcgccgc tacagggcgc gtcccattcg 4620

<210> 45
<211> 6298
<212> DNA
<213> Artificial sequence

<220>
<223> Synthetic oligonucleotide


<220>
<221> misc_feature
<222> (428)..(2161)
<223> n is a, c, g, or t

<400> 45
ccattcaggc tgcgcaactg ttgggaaggg cgatcggtgc gggcctcttc gctattacgc 60

cagctggcga aagggggatg tgctgcaagg cgattaagtt gggtaacgcc agggttttcc 120

cagtcacgac gttgtaaaac gacggccagt gaattgaatt taggtgacac tatagaagag 180

ctatgacgtc gcatgcaatt aaccctcact aaagggacgc gtacgtaagc ttggatcctc 240

tagagcggcc gccggactcc catggggacc aggacatgtg tcacgcagct taaattcgca 300
ctctcgtaat atagacacga cgctctcata atagaggcgc acatggtata ttatgctcgc 360
acctgctata ttgagcggca cattattact gacatcgaac tgctacgact gacacgacca 420
tctcgagnnn nnnnnnnnnn nnnnnnnnnn nnnnnnnnnn nnnnnnnnnn nnnnnnnnnn 480
nnnnnnnnnn nnnnnnnnnn nnnnnnnnnn nnnnnnnnnn nnnnnnnnnn nnnnnnnnnn 540
nnnnnnnnnn nnnnnnnnnn nnnnnnnnnn nnnnnnnnnn nnnnnnnnnn nnnnnnnnnn 600
nnnnnnnnnn nnnnnnnnnn nnnnnnnnnn nnnnnnnnnn nnnnnnnnnn nnnnnnnnnn 660
nnnnnnnnnn nnnnnnnnnn nnnnnnnnnn nnnnnnnnnn nnnnnnnnnn nnnnnnnnnn 720
nnnnnnnnnn nnnnnnnnnn nnnnnnnnnn nnnnnnnnnn nnnnnnnnnn nnnnnnnnnn 780
nnnnnnnnnn nnnnnnnnnn nnnnnnnnnn nnnnnnnnnn nnnnnnnnnn nnnnnnnnnn 840
nnnnnnnnnn nnnnnnnnnn nnnnnnnnnn nnnnnnnnnn nnnnnnnnnn nnnnnnnnnn 900
nnnnnnnnnn nnnnnnnnnn nnnnnnnnnn nnnnnnnnnn nnnnnnnnnn nnnnnnnnnn 960
nnnnnnnnnn nnnnnnnnnn nnnnnnnnnn nnnnnnnnnn nnnnnnnnnn nnnnnnnnnn 1020
nnnnnnnnnn nnnnnnnnnn nnnnnnnnnn nnnnnnnnnn nnnnnnnnnn nnnnnnnnnn 1080
nnnnnnnnnn nnnnnnnnnn nnnnnnnnnn nnnnnnnnnn nnnnnnnnnn nnnnnnnnnn 1140
nnnnnnnnnn nnnnnnnnnn nnnnnnnnnn nnnnnnnnnn nnnnnnnnnn nnnnnnnnnn 1200
nnnnnnnnnn nnnnnnnnnn nnnnnnnnnn nnnnnnnnnn nnnnnnnnnn nnnnnnnnnn 1260
nnnnnnnnnn nnnnnnnnnn nnnnnnnnnn nnnnnnnnnn nnnnnnnnnn nnnnnnnnnn 1320
nnnnnnnnnn nnnnnnnnnn nnnnnnnnnn nnnnnnnnnn nnnnnnnnnn nnnnnnnnnn 1380
nnnnnnnnnn nnnnnnnnnn nnnnnnnnnn nnnnnnnnnn nnnnnnnnnn nnnnnnnnnn 1440
nnnnnnnnnn nnnnnnnnnn nnnnnnnnnn nnnnnnnnnn nnnnnnnnnn nnnnnnnnnn 1500
nnnnnnnnnn nnnnnnnnnn nnnnnnnnnn nnnnnnnnnn nnnnnnnnnn nnnnnnnnnn 1560
nnnnnnnnnn nnnnnnnnnn nnnnnnnnnn nnnnnnnnnn nnnnnnnnnn nnnnnnnnnn 1620
nnnnnnnnnn nnnnnnnnnn nnnnnnnnnn nnnnnnnnnn nnnnnnnnnn nnnnnnnnnn 1680
nnnnnnnnnn nnnnnnnnnn nnnnnnnnnn nnnnnnnnnn nnnnnnnnnn nnnnnnnnnn 1740
nnnnnnnnnn nnnnnnnnnn nnnnnnnnnn nnnnnnnnnn nnnnnnnnnn nnnnnnnnnn 1800
nnnnnnnnnn nnnnnnnnnn nnnnnnnnnn nnnnnnnnnn nnnnnnnnnn nnnnnnnnnn 1860
nnnnnnnnnn nnnnnnnnnn nnnnnnnnnn nnnnnnnnnn nnnnnnnnnn nnnnnnnnnn 1920
nnnnnnnnnn nnnnnnnnnn nnnnnnnnnn nnnnnnnnnn nnnnnnnnnn nnnnnnnnnn 1980
nnnnnnnnnn nnnnnnnnnn nnnnnnnnnn nnnnnnnnnn nnnnnnnnnn nnnnnnnnnn 2040
nnnnnnnnnn nnnnnnnnnn nnnnnnnnnn nnnnnnnnnn nnnnnnnnnn nnnnnnnnnn 2100
nnnnnnnnnn nnnnnnnnnn nnnnnnnnnn nnnnnnnnnn nnnnnnnnnn nnnnnnnnnn 2160
ngctagcgcc atcgtactca gatatcaagc tcctcatcag taatccattc acgacctatg 2220
agacgcagtt cacacatcat aacacgcgac cagacaggcc acgcagtgct agaggataca 2280

cacccatgat acggtagtcg tctactgcta gtccatgcgt catgctaagt cgtcgtagta 2340
gatctccaag ggactcacat gtattaatgg accgcagcgt caccaggtcg acgaattccg 2400
gaaaaaaaaa aaaaaaaaaa aactgcagtc tagaaagctt ctgcagggcg cgccatttaa 2460
attgcaggcg taccagcttt ccctatagtg agtcgtatta gagcttggcg taatcatggt 2520
catagctgtt tcctgtgtga aattgttatc cgctcacaat tccacacaac atacgagccg 2580
gaagcataaa gtgtaaagcc tggggtgcct aatgagtgag ctaactcaca ttaattgcgt 2640
tgcgctcact gcccgctttc cagtcgggaa acctgtcgtg ccagctgcat taatgaatcg 2700
gccaacgcgc ggggagaggc ggtttgcgta ttgggcgcca gggtggtttt tcttttcacc 2760
agtgagacgg gcaacagctg attgcccttc accgcctggc cctgagagag ttgcagcaag 2820
cggtccacgc tggtttgccc cagcaggcga aaatcctgtt tgatggtggt taacggcggg 2880
atataacatg agctgtcttc ggtatcgtcg tatcccacta ccgagatatc cgcaccaacg 2940
cgcagcccgg actcggtaat ggcgcgcatt gcgcccagcg ccatctgatc gttggcaacc 3000
agcatcgcag tgggaacgat gccctcattc agcatttgca tggtttgttg aaaaccggac 3060
atggcactcc agtcgccttc ccgttccgct atcggctgaa tttgattgcg agtgagatat 3120
ttatgccagc cagccagacg cagacgcgcc gagacagaac ttaatgggcc cgctaacagc 3180
gcgatttgct ggtgacccaa tgcgaccaga tgctccacgc ccagtcgcgt accgtcttca 3240
tgggagaaaa taatactgtt gatgggtgtc tggtcagaga catcaagaaa taacgccgga 3300
acattagtgc aggcagcttc cacagcaatg gcatcctggt catccagcgg atagttaatg 3360
atcagcccac tgacccgttg cgcgagaaga ttgtgcaccg ccgctttaca ggcttcgacg 3420
ccgcttcgtt ctaccatcga caccaccacg ctggcaccca gttgatcggc gcgagattta 3480
atcgccgcga caatttgcga cggcgcgtgc agggccagac tggaggtggc aacgccaatc 3540
agcaacgact gtttgcccgc cagttgttgt gccacgcggt tgggaatgta attcagctcc 3600
gccatcgccg cttccacttt ttcccgcgtt ttcgcagaaa cgtggctggc ctggttcacc 3660
acgcgggaaa cggtctgata agagacaccg gcatactctg cgacatcgta taacgttact 3720
ggtttcacat tcaccaccct gaattgactc tcttccgggc gctatcatgc cataccgcga 3780
aaggttttgc gccattcgat ggtgtcaacg taaatgccgc ttcgccttcg cgcgcgaatt 3840
gcaagctctg cattaatgaa tcggccaacg cgcggggaga ggcggtttgc gtattgggcg 3900
ctcttccgct tcctcgctca ctgactcgct gcgctcggtc gttcggctgc ggcgagcggt 3960
atcagctcac tcaaaggcgg taatacggtt atccacagaa tcaggggata acgcaggaaa 4020
gaacatgtga gcaaaaggcc agcaaaaggc caggaaccgt aaaaaggccg cgttgctggc 4080
gtttttccat aggctccgcc cccctgacga gcatcacaaa aatcgacgct caagtcagag 4140
gtggcgaaac ccgacaggac tataaagata ccaggcgttt ccccctggaa gctccctcgt 4200
gcgctctcct gttccgaccc tgccgcttac cggatacctg tccgcctttc tcccttcggg 4260
aagcgtggcg ctttctcaat gctcacgctg taggtatctc agttcggtgt aggtcgttcg 4320

ctccaagctg ggctgtgtgc acgaaccccc cgttcagccc gaccgctgcg ccttatccgg 4380
taactatcgt cttgagtcca acccggtaag acacgactta tcgccactgg cagcagccac 4440
tggtaacagg attagcagag cgaggtatgt aggcggtgct acagagttct tgaagtggtg 4500
gcctaactac ggctacacta gaaggacagt atttggtatc tgcgctctgc tgaagccagt 4560
taccttcgga aaaagagttg gtagctcttg atccggcaaa caaaccaccg ctggtagcgg 4620
tggttttttt gtttgcaagc agcagattac gcgcagaaaa aaaggatctc aagaagatcc 4680
tttgatcttt tctacggggt ctgacgctca gtggaacgaa aactcacgtt aagggatttt 4740
ggtcatgaga ttatcaaaaa ggatcttcac ctagatcctt ttaaattaaa aatgaagttt 4800
taaatcaatc taaagtatat atgagtaaac ttggtctgac agttaccaat gcttaatcag 4860
tgaggcacct atctcagcga tctgtctatt tcgttcatcc atagttgcct gactccccgt 4920
cgtgtagata actacgatac gggagggctt accatctggc cccagtgctg caatgatacc 4980
gcgagaccca cgctcaccgg ctccagattt atcagcaata aaccagccag ccggaagggc 5040
cgagcgcaga agtggtcctg caactttatc cgcctccatc cagtctatta attgttgccg 5100
ggaagctaga gtaagtagtt cgccagttaa tagtttgcgc aacgttgttg ccattgctac 5160
aggcatcgtg gtgtcacgct cgtcgtttgg tatggcttca ttcagctccg gttcccaacg 5220
atcaaggcga gttacatgat cccccatgtt gtgcaaaaaa gcggttagct ccttcggtcc 5280
tccgatcgtt gtcagaagta agttggccgc agtgttatca ctcatggtta tggcagcact 5340
gcataattct cttactgtca tgccatccgt aagatgcttt tctgtgactg gtgagtactc 5400
aaccaagtca ttctgagaat agtgtatgcg gcgaccgagt tgctcttgcc cggcgtcaat 5460
acgggataat accgcgccac atagcagaac tttaaaagtg ctcatcattg gaaaacgttc 5520
ttcggggcga aaactctcaa ggatcttacc gctgttgaga tccagttcga tgtaacccac 5580
tcgtgcaccc aactgatctt cagcatcttt tactttcacc agcgtttctg ggtgagcaaa 5640
aacaggaagg caaaatgccg caaaaaaggg aataagggcg acacggaaat gttgaatact 5700
catactcttc ctttttcaat attattgaag catttatcag ggttattgtc tcatgagcgg 5760
atacatattt gaatgtattt agaaaaataa acaaataggg gttccgcgca catttccccg 5820
aaaagtgcca cctgaaattg taaacgttaa tattttgtta aaattcgcgt taaatttttg 5880
ttaaatcagc tcatttttta accaataggc cgaaatcggc aaaatccctt ataaatcaaa 5940
agaatagacc gagatagggt tgagtgttgt tccagtttgg aacaagagtc cactattaaa 6000
gaacgtggac tccaacgtca aagggcgaaa aaccgtctat cagggcgatg gcccactacg 6060
tgaaccatca ccctaatcaa gttttttggg gtcgaggtgc cgtaaagcac taaatcggaa 6120
ccctaaaggg agcccccgat ttagagcttg acggggaaag ccggcgaacg tggcgagaaa 6180
ggaagggaag aaagcgaaag gagcgggcgc tagggcgctg gcaagtgtag cggtcacgct 6240
gcgcgtaacc accacacccg ccgcgcttaa tgcgccgcta cagggcgcgt cccattcg 6298

<210> 46

<211>  4610
<212>  DNA
<213>  Artificial sequence

<220>
<223>  Synthetic oligonucleotide

<400>  46
ccattcaggc tgcgcaactg ttgggaaggg cgatcggtgc gggcctcttc gctattacgc      60

cagctggcga aagggggatg tgctgcaagg cgattaagtt gggtaacgcc agggttttcc     120

cagtcacgac gttgtaaaac gacggccagt gaattgaatt taggtgacac tatagaagag     180

ctatgacgtc gcatgcaatt aaccctcact aaagggacgc gtacgtaagc ttggatcctc     240

tagagcggcc gccggactcc catggggacc aggacatgtg tcacgcagct taaattcgca     300

ctctcgtaat atagacacga cgctctcata atagaggcgc acatggtata ttatgctcgc     360

acctgctata ttgagcggca cattattact gacatcgaac tgctacgact gacacgacca     420

tctcgagact agtcgttatc aacctgcgtc cgagcagtaa tttcaatcgc gtggctagcg     480

ccatcgtact cagatatcaa gctcctcatc agtaatccat tcacgaccta tgagacgcag     540

ttcacacatc ataacacgcg accagacagg ccacgcagtg ctagaggata cacacccatg     600

atacggtagt cgtctactgc tagtccatgc gtcatgctaa gtcgtcgtag tagatctcca     660

agggactcac atgtattaat ggaccgcagc gtcaccaggt cgacgaattc cggaaaaaaa     720

aaaaaaaaaa aaaactgcag tctagaaagc ttctgcaggg cgcgccattt aaattgcagg     780

cgtaccagct ttccctatag tgagtcgtat tagagcttgg cgtaatcatg gtcatagctg     840

tttcctgtgt gaaattgtta tccgctcaca attccacaca acatacgagc cggaagcata     900

aagtgtaaag cctggggtgc ctaatgagtg agctaactca cattaattgc gttgcgctca     960

ctgcccgctt tccagtcggg aaacctgtcg tgccagctgc attaatgaat cggccaacgc    1020

gcggggagag gcggtttgcg tattgggcgc cagggtggtt tttcttttca ccagtgagac    1080

gggcaacagc tgattgccct tcaccgcctg gccctgagag agttgcagca agcggtccac    1140

gctggtttgc cccagcaggc gaaaatcctg tttgatggtg gttaacggcg ggatataaca    1200

tgagctgtct tcggtatcgt cgtatcccac taccgagata tccgcaccaa cgcgcagccc    1260

ggactcggta atggcgcgca ttgcgcccag cgccatctga tcgttggcaa ccagcatcgc    1320

agtgggaacg atgccctcat tcagcatttg catggtttgt tgaaaaccgg acatggcact    1380

ccagtcgcct tcccgttccg ctatcggctg aatttgattg cgagtgagat atttatgcca    1440

gccagccaga cgcagacgcg ccgagacaga acttaatggg cccgctaaca gcgcgatttg    1500

ctggtgaccc aatgcgacca gatgctccac gcccagtcgc gtaccgtctt catgggagaa    1560

aataatactg ttgatgggtg tctggtcaga gacatcaaga aataacgccg gaacattagt    1620

gcaggcagct tccacagcaa tggcatcctg gtcatccagc ggatagttaa tgatcagccc    1680

actgacccgt tgcgcgagaa gattgtgcac cgccgcttta caggcttcga cgccgcttcg    1740

ttctaccatc gacaccacca cgctggcacc cagttgatcg gcgcgagatt taatcgccgc    1800

gacaatttgc gacggcgcgt gcagggccag actggaggtg gcaacgccaa tcagcaacga 1860
ctgtttgccc gccagttgtt gtgccacgcg gttgggaatg taattcagct ccgccatcgc 1920
cgcttccact ttttcccgcg ttttcgcaga aacgtggctg gcctggttca ccacgcggga 1980
aacggtctga taagagacac cggcatactc tgcgacatcg tataacgtta ctggtttcac 2040
attcaccacc ctgaattgac tctcttccgg gcgctatcat gccataccgc gaaaggtttt 2100
gcgccattcg atggtgtcaa cgtaaatgcc gcttcgcctt cgcgcgcgaa ttgcaagctc 2160
tgcattaatg aatcggccaa cgcgcgggga gaggcggttt gcgtattggg cgctcttccg 2220
cttcctcgct cactgactcg ctgcgctcgg tcgttcggct gcggcgagcg gtatcagctc 2280
actcaaaggc ggtaatacgg ttatccacag aatcagggga taacgcagga aagaacatgt 2340
gagcaaaagg ccagcaaaag gccaggaacc gtaaaaaggc cgcgttgctg gcgtttttcc 2400
ataggctccg cccccctgac gagcatcaca aaaatcgacg ctcaagtcag aggtggcgaa 2460
acccgacagg actataaaga taccaggcgt ttccccctgg aagctccctc gtgcgctctc 2520
ctgttccgac cctgccgctt accggatacc tgtccgcctt tctcccttcg ggaagcgtgg 2580
cgctttctca atgctcacgc tgtaggtatc tcagttcggt gtaggtcgtt cgctccaagc 2640
tgggctgtgt gcacgaaccc cccgttcagc ccgaccgctg cgccttatcc ggtaactatc 2700
gtcttgagtc caacccggta agacacgact tatcgccact ggcagcagcc actggtaaca 2760
ggattagcag agcgaggtat gtaggcggtg ctacagagtt cttgaagtgg tggcctaact 2820
acggctacac tagaaggaca gtatttggta tctgcgctct gctgaagcca gttaccttcg 2880
gaaaaagagt tggtagctct tgatccggca aacaaaccac cgctggtagc ggtggttttt 2940
ttgtttgcaa gcagcagatt acgcgcagaa aaaaaggatc tcaagaagat cctttgatct 3000
tttctacggg gtctgacgct cagtggaacg aaaactcacg ttaagggatt ttggtcatga 3060
gattatcaaa aaggatcttc acctagatcc ttttaaatta aaaatgaagt tttaaatcaa 3120
tctaaagtat atatgagtaa acttggtctg acagttacca atgcttaatc agtgaggcac 3180
ctatctcagc gatctgtcta tttcgttcat ccatagttgc ctgactcccc gtcgtgtaga 3240
taactacgat acgggagggc ttaccatctg gccccagtgc tgcaatgata ccgcgagacc 3300
cacgctcacc ggctccagat ttatcagcaa taaaccagcc agccggaagg gccgagcgca 3360
gaagtggtcc tgcaacttta tccgcctcca tccagtctat taattgttgc cgggaagcta 3420
gagtaagtag ttcgccagtt aatagtttgc gcaacgttgt tgccattgct acaggcatcg 3480
tggtgtcacg ctcgtcgttt ggtatggctt cattcagctc cggttcccaa cgatcaaggc 3540
gagttacatg atcccccatg ttgtgcaaaa aagcggttag ctccttcggt cctccgatcg 3600
ttgtcagaag taagttggcc gcagtgttat cactcatggt tatggcagca ctgcataatt 3660
ctcttactgt catgccatcc gtaagatgct tttctgtgac tggtgagtac tcaaccaagt 3720
cattctgaga atagtgtatg cggcgaccga gttgctcttg cccggcgtca atacgggata 3780
ataccgcgcc acatagcaga actttaaaag tgctcatcat tggaaaacgt tcttcggggc 3840

gaaaactctc aaggatctta ccgctgttga gatccagttc gatgtaaccc actcgtgcac 3900
ccaactgatc ttcagcatct tttactttca ccagcgtttc tgggtgagca aaaacaggaa 3960
ggcaaaatgc cgcaaaaaag ggaataaggg cgacacggaa atgttgaata ctcatactct 4020
tcctttttca atattattga agcatttatc agggttattg tctcatgagc ggatacatat 4080
ttgaatgtat ttagaaaaat aaacaaatag gggttccgcg cacatttccc cgaaaagtgc 4140
cacctgaaat tgtaaacgtt aatattttgt taaaattcgc gttaaatttt tgttaaatca 4200
gctcattttt taaccaatag gccgaaatcg gcaaaatccc ttataaatca aaagaataga 4260
ccgagatagg gttgagtgtt gttccagttt ggaacaagag tccactatta aagaacgtgg 4320
actccaacgt caaagggcga aaaaccgtct atcagggcga tggcccacta cgtgaaccat 4380
caccctaatc aagttttttg gggtcgaggt gccgtaaagc actaaatcgg aaccctaaag 4440
ggagcccccg atttagagct tgacggggaa agccggcgaa cgtggcgaga aaggaaggga 4500
agaaagcgaa aggagcgggc gctagggcgc tggcaagtgt agcggtcacg ctgcgcgtaa 4560
ccaccacacc cgccgcgctt aatgcgccgc tacagggcgc gtcccattcg 4610

<210> 47
<211> 23
<212> DNA
<213> Artificial sequence

<220>
<223> Synthetic oligonucleotide

<400> 47
ccaagatagt cagctccgaa cca 23

<210> 48
<211> 20
<212> DNA
<213> Artificial sequence

<220>
<223> Synthetic oligonucleotide

<400> 48
gcggcagcga catacacaca 20

<210> 49
<211> 25
<212> DNA
<213> Artificial sequence

<220>
<223> Synthetic oligonucleotide

<400> 49
tgagagcatg atgtaactag agcgc 25

<210> 50
<211> 25
<212> DNA
<213> Artificial sequence

<220>
<223>  Synthetic oligonucleotide

<400>  50
cgcacacatt gatacaaccg tctac                                          25


<210>  51
<211>  25
<212>  DNA
<213>  Artificial sequence

<220>
<223>  Synthetic oligonucleotide

<400>  51
ccacagatga agataccctc acaca                                          25


<210>  52
<211>  25
<212>  DNA
<213>  Artificial sequence

<220>
<223>  Synthetic oligonucleotide

<400>  52
cgccactaat caactggact aagct                                          25


<210>  53
<211>  23
<212>  DNA
<213>  Artificial sequence

<220>
<223>  Synthetic oligonucleotide

<400>  53
cctcacgatt cgataaatga cgc                                            23


<210>  54
<211>  25
<212>  DNA
<213>  Artificial sequence

<220>
<223>  Synthetic oligonucleotide

<400>  54
cgcccgtagc acagatataa ctcac                                          25


<210>  55
<211>  23
<212>  DNA
<213>  Artificial sequence

<220>
<223>  Synthetic oligonucleotide

<400>  55
cacgcgcacg agatacagta cac                                            23


<210>  56

<211>  24
<212>  DNA
<213>  Artificial sequence

<220>
<223>  Synthetic oligonucleotide

<400>  56
cggcgagaga cgtaatcaga tcac                                              24


<210>  57
<211>  23
<212>  DNA
<213>  Artificial sequence

<220>
<223>  Synthetic oligonucleotide

<400>  57
cagaccgaga taaggtgcga tca                                               23


<210>  58
<211>  25
<212>  DNA
<213>  Artificial sequence

<220>
<223>  Synthetic oligonucleotide

<400>  58
cggcagtatg aatagtaagc ccatg                                             25


<210>  59
<211>  23
<212>  DNA
<213>  Artificial sequence

<220>
<223>  Synthetic oligonucleotide

<400>  59
gcgataggcc atgatatgaa cgt                                               23


<210>  60
<211>  24
<212>  DNA
<213>  Artificial sequence

<220>
<223>  Synthetic oligonucleotide

<400>  60
ggacactcct agcaggtagc acga                                              24


<210>  61
<211>  24
<212>  DNA
<213>  Artificial sequence

<220>
<223>  Synthetic oligonucleotide

<400>  61

cgcagcgtca ataactatag ccac 24

<210> 62
<211> 19
<212> DNA
<213> Artificial sequence

<220>
<223> Synthetic oligonucleotide

<400> 62
agcccggcgt agaaagcat 19

<210> 63
<211> 21
<212> DNA
<213> Artificial sequence

<220>
<223> Synthetic oligonucleotide

<400> 63
ggaaatcatt acacgccggt g 21

<210> 64
<211> 23
<212> DNA
<213> Artificial sequence

<220>
<223> Synthetic oligonucleotide

<400> 64
cgacaatagt atcaccgcgt cgt 23

<210> 65
<211> 23
<212> DNA
<213> Artificial sequence

<220>
<223> Synthetic oligonucleotide

<400> 65
agtcagaccg cgacctcaat aag 23

<210> 66
<211> 25
<212> DNA
<213> Artificial sequence

<220>
<223> Synthetic oligonucleotide

<400> 66
gcaagacccc gctaggttaa tagtc 25

<210> 67
<211> 20
<212> DNA
<213> Artificial sequence

<220>
<223>  Synthetic oligonucleotide

<400>  67
tgctcggacg caggttgata                                                    20


<210>  68
<211>  25
<212>  DNA
<213>  Artificial sequence

<220>
<223>  Synthetic oligonucleotide

<400>  68
gtggaaacta cctcactcac tcagg                                              25


<210>  69
<211>  22
<212>  DNA
<213>  Artificial sequence

<220>
<223>  Synthetic oligonucleotide

<400>  69
gtggaactat tcgcaggtac gc                                                 22


<210>  70
<211>  25
<212>  DNA
<213>  Artificial sequence

<220>
<223>  Synthetic oligonucleotide

<400>  70
agtagtctac gacgtgtcta aggcg                                              25


<210>  71
<211>  25
<212>  DNA
<213>  Artificial sequence

<220>
<223>  Synthetic oligonucleotide

<400>  71
tcgctctaca gtggtataag accga                                              25


<210>  72
<211>  22
<212>  DNA
<213>  Artificial sequence

<220>
<223>  Synthetic oligonucleotide

<400>  72
catcgcctca ggtgattatc ca                                                 22


<210>  73

<211>  24
<212>  DNA
<213>  Artificial sequence

<220>
<223>  Synthetic oligonucleotide

<400>  73
cgtgtcggtc agagtggtaa catc                                              24


<210>  74
<211>  21
<212>  DNA
<213>  Artificial sequence

<220>
<223>  Synthetic oligonucleotide

<400>  74
catcggcgcg tatggtaaca t                                                 21


<210>  75
<211>  23
<212>  DNA
<213>  Artificial sequence

<220>
<223>  Synthetic oligonucleotide

<400>  75
gctatatgcg ccgtgtgaaa cat                                               23


<210>  76
<211>  24
<212>  DNA
<213>  Artificial sequence

<220>
<223>  Synthetic oligonucleotide

<400>  76
caggttctgc cgatgtgagt atct                                              24


<210>  77
<211>  23
<212>  DNA
<213>  Artificial sequence

<220>
<223>  Synthetic oligonucleotide

<400>  77
gatgttcatc cgtgcgtaga atg                                               23


<210>  78
<211>  24
<212>  DNA
<213>  Artificial sequence

<220>
<223>  Synthetic oligonucleotide

<400>  78

cgcttagtac agtctcatgc gagg 24

<210> 79
<211> 23
<212> DNA
<213> Artificial sequence

<220>
<223> Synthetic oligonucleotide

<400> 79
cagtcatatc cgcgattgaa acc 23

<210> 80
<211> 25
<212> DNA
<213> Artificial sequence

<220>
<223> Synthetic oligonucleotide

<400> 80
gtgtatagag ttctgctggt gcgat 25

<210> 81
<211> 24
<212> DNA
<213> Artificial sequence

<220>
<223> Synthetic oligonucleotide

<400> 81
cgtatggcat tgtactcgca gagt 24

<210> 82
<211> 25
<212> DNA
<213> Artificial sequence

<220>
<223> Synthetic oligonucleotide

<400> 82
ctgctatgca cgtctactta cacga 25

<210> 83
<211> 24
<212> DNA
<213> Artificial sequence

<220>
<223> Synthetic oligonucleotide

<400> 83
gtcagcgtag cacgtcgtac atag 24

<210> 84
<211> 21
<212> DNA
<213> Artificial sequence

<220>
<223>  Synthetic oligonucleotide

<400>  84
cgggctacag cgtcgaatta g                                                21


<210>  85
<211>  21
<212>  DNA
<213>  Artificial sequence

<220>
<223>  Synthetic oligonucleotide

<400>  85
cgggaatacc ttatgcacgg g                                                21


<210>  86
<211>  21
<212>  DNA
<213>  Artificial sequence

<220>
<223>  Synthetic oligonucleotide

<400>  86
gcgaatatac tgtgccagcg g                                                21


<210>  87
<211>  40
<212>  DNA
<213>  Artificial sequence

<220>
<223>  Synthetic oligonucleotide

<400>  87
tgacattatt ggttcggagc tgactatctt ggtgtcgcgc                            40


<210>  88
<211>  40
<212>  DNA
<213>  Artificial sequence

<220>
<223>  Synthetic oligonucleotide

<400>  88
taccgctgtg tgtatgtcgc tgccgctctt tatgctacgg                            40

## Description

### RELATED APPLICATIONS

This application claims the benefit of U.S. Provisional Application No. 60/610,668 filed September 17, 2004, the entire disclosure of which is incorporated herein by reference.

### FIELD OF THE INVENTION

The present invention relates to the field of nucleic acid analysis and to methods for marking samples with an internal detectable marking system.

### BACKGROUND OF THE INVENTION

Methods of genetic analysis of biological samples typically involve analysis of a liquid aliquot of the sample. The aliquot for analysis is typically transferred from the original source to a new container for subsequent analysis. The new container is typically associated with the original sample and the original source, for example, by a labeling system, whereby the containers are labeled. The movement of an aliquot of a sample to a new container presents an opportunity for the aliquot to be wrongly associated, for example, if the new container is not labeled correctly. Aliquoting and subsequent manipulation steps are also opportunities for introduction of contamination to the sample, for example, mixing of material from two different biological sources.

### SUMMARY OF THE INVENTION

Methods for marking biological samples with barcodes are disclosed. Barcodes are generated by combining barcode plasmids or barcode adaptors in combinations of two or more so that samples within a set of samples are uniquely marked. The barcode sequences may be carried on plasmids so that fragments containing the barcode sequence may be released by restriction digestion into fragments that can be ligated to adaptors comprising priming sites and amplified, for example, by PCR.

In preferred embodiments barcodes are amplified in the sample in parallel with the amplification of the marked sample. For example, if the sample is being genotyped the barcode sequences are amplified by the same method with which the genomic fragments for genotyping are analyzed. In preferred embodiments this is by the WGSA method of fragmentation with a restriction enzyme, adaptor ligation and amplification of fragments of a selected, limited size range. If the sample is being analyzed for gene expression analysis the barcode may be present as a polyadenylated transcript for amplification by a T7 oligodT primer or without a polyA tail for amplification by random primers with or without T7.

Kits comprising marker molecules, including barcode plasmids and barcode adaptors are disclosed. The marker molecules may be arranged in a format that facilitates barcoding, for example a multiwell plate.

The methods are particularly useful for detection of contamination of one sample by another sample, mis-identification of samples and contamination of one sample with amplicons of another sample.

### BRIEF DESCRIPTION OF THE FIGURES

Figure 1 shows a schematic of one embodiment. A plasmid containing a barcode sequence is fragmented with a selected restriction enzyme. The barcode sequence is contained within a fragment that is between 250 and 2000 base pairs. Adaptors are ligated to the fragments and the fragment containing the barcode is efficiently amplified.
Figure 2 shows an example of a possible arrangement of the barcode probe sets on an array. A probe set that is complementary to each barcode is present at two different locations on the array and the barcode probe sets are positioned throughout the array. Visual inspection of the hybridization pattern can distinguish between different barcode combinations.
Figure 3 shows a schematic of a method of simultaneously detecting a fragment of interest (103) and a tag sequence in a marker molecule (105).
Figure 4 is a schematic of fragments that are expected when barcode sequences are added as barcode fragments that can be ligated to adaptors.

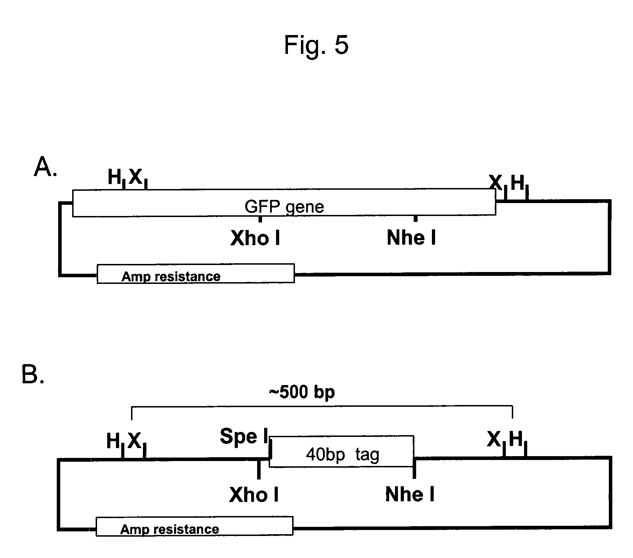

Figures 5A and 5B show a schematic of the 100K barcode plasmids. Figure 5A shows the pFC48 vector, and 5B shows the 100K clones.

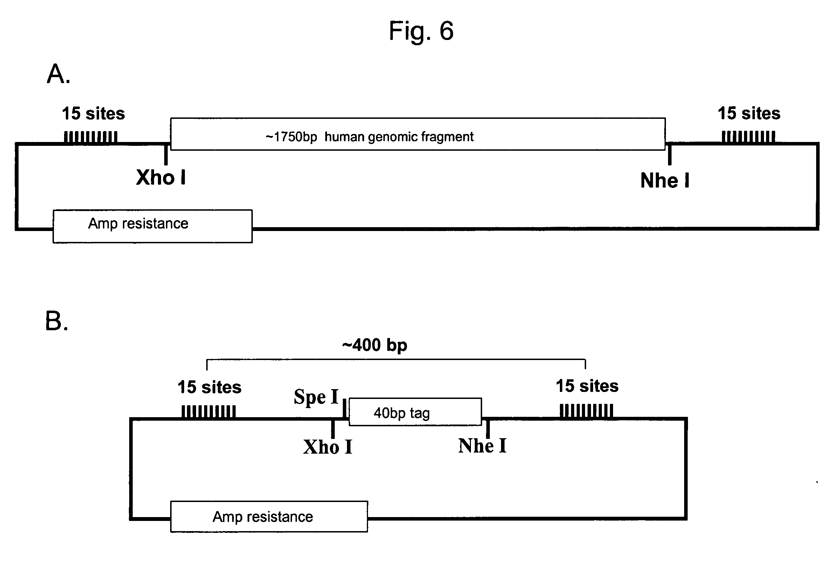

Figures 6A and 6B show a schematic of the 500K barcode plasmids. Figure 6A shows the pFC51 vector, and 6B shows the 500K clones.

### DETAILED DESCRIPTION OF THE INVENTION

### a) General

The present invention has many preferred embodiments and relies on many patents, applications and other references for details known to those of the art. Therefore, when a patent, application, or other reference is cited or repeated below, it should be understood that it is incorporated by reference in its entirety for all purposes as well as for the proposition that is recited.

As used in this application, the singular form "a," "an," and "the" include plural references unless the context clearly dictates otherwise. For example, the term "an agent" includes a plurality of agents, including mixtures thereof.

An individual is not limited to a human being but may also be other organisms including but not limited to mammals, plants, bacteria, or cells derived from any of the above.

Throughout this disclosure, various aspects of this invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

The practice of the present invention may employ, unless otherwise indicated, conventional techniques and descriptions of organic chemistry, polymer technology, molecular biology (including recombinant techniques), cell biology, biochemistry, and immunology, which are within the skill of the art. Such conventional techniques include polymer array synthesis, hybridization, ligation, and detection of hybridization using a label. Specific illustrations of suitable techniques can be had by reference to the example herein below. However, other equivalent conventional procedures can, of course, also be used. Such conventional techniques and descriptions can be found in standard laboratory manuals such as *Genome Analysis: A Laboratory Manual Series (Vols. I-IV), Using Antibodies: A Laboratory Manual, Cells: A Laboratory Manual, PCR Primer: A Laboratory Manual,* and Molecular *Cloning: A Laboratory Manual* (all from Cold Spring Harbor Laboratory Press), Stryer, L. (1995) *Biochemistry* (4th Ed.) Freeman, New York, *Gait, "Oligonucleotide Synthesis: A Practical Approach" 1984, IRL Press, London,* Nelson and Cox (2000), *Lehninger, Principles of Biochemistry* 3^{rd} Ed., W.H. Freeman Pub., New York, NY and Berg et al. (2002) *Biochemistry,* 5^{th} Ed., W.H. Freeman Pub., New York, NY, all of which are herein incorporated in their entirety by reference for all purposes.

The present invention can employ solid substrates, including arrays in some preferred embodiments. Methods and techniques applicable to polymer (including protein) array synthesis have been described in U.S. Serial No. 09/536,841, WO 00/58516, U.S. Patent Nos. 5,143,854, 5,242,974, 5,252,743, 5,324,633, 5,384,261, 5,405,783, 5,424,186, 5,451,683, 5,482,867, 5,491,074, 5,527,681, 5,550,215, 5,571,639, 5,578,832, 5,593,839, 5,599,695, 5,624,711, 5,631,734, 5,795,716, 5,831,070, 5,837,832, 5,856,101, 5,858,659, 5,936,324, 5,968,740, 5,974,164, 5,981,185, 5,981,956, 6,025,601, 6,033,860, 6,040,193, 6,090,555, 6,136,269, 6,269,846 and 6,428,752, in PCT Applications Nos. PCT/US99/00730 (International Publication No. WO 99/36760) and PCT/US01/04285 (International Publication No. WO 01/58593), which are all incorporated herein by reference in their entirety for all purposes.

Patents that describe synthesis techniques in specific embodiments include U.S. Patent Nos. 5,412,087, 6,147,205, 6,262,216, 6,310,189, 5,889,165, and 5,959,098. Nucleic acid arrays are described in many of the above patents, but the same techniques are applied to polypeptide arrays.

Nucleic acid arrays that are useful in the present invention include those that are commercially available from Affymetrix (Santa Clara, CA) under the brand name GeneChip®. Example arrays are shown on the website at affymetrix.com.

The present invention also contemplates many uses for polymers attached to solid substrates. These uses include gene expression monitoring, profiling, library screening, genotyping and diagnostics. Gene expression monitoring and profiling methods can be shown in U.S. Patent Nos. 5,800,992, 6,013,449, 6,020,135, 6,033,860, 6,040,138, 6,177,248 and 6,309,822. Genotyping and uses therefore are shown in U.S. Serial Nos. 10/442,021, 10/013,598 (U.S. Patent Application Publication 20030036069), and U.S. Patent Nos. 5,856,092, 6,300,063, 5,858,659, 6,284,460, 6,361,947, 6,368,799 and 6,333,179. Other uses are embodied in U.S. Patent Nos. 5,871,928, 5,902,723, 6,045,996, 5,541,061, and 6,197,506.

The present invention also contemplates sample preparation methods in certain preferred embodiments. Prior to or concurrent with genotyping, the genomic sample may be amplified by a variety of mechanisms, some of which may employ PCR. *See,* for example, *PCR Technology: Principles and Applications for DNA Amplification* (Ed. H.A. Erlich, Freeman Press, NY, NY, 1992); *PCR Protocols: A Guide to Methods and Applications* (Eds. Innis, et al., Academic Press, San Diego, CA, 1990); Mattila et al., *Nucleic Acids Res.* 19, 4967 (1991); Eckert et al., *PCR Methods and Applications* 1, 17 (1991); *PCR* (Eds. McPherson et al., IRL Press, Oxford); and U.S. Patent Nos. 4,683,202, 4,683,195, 4,800,159 4,965,188,and 5,333,675, each of which is incorporated herein by reference in its entirety for all purposes. The sample may be amplified on the array. See, for example, U.S. Patent No. 6,300,070 and U.S. Serial No. 09/513,300, which are incorporated herein by reference.

Other suitable amplification methods include the ligase chain reaction (LCR) *(for example,* Wu and Wallace, *Genomics* 4, 560 (1989), Landegren et al., *Science* 241, 1077 (1988) and Barringer et al. *Gene* 89:117 (1990)), transcription amplification (Kwoh et al., *Proc. Natl. Acad. Sci. USA* 86, 1173 (1989) and W088/10315), self-sustained sequence replication (Guatelli et al., *Proc. Nat. Acad. Sci. USA,* 87, 1874 (1990) and WO90/06995), selective amplification of target polynucleotide sequences (U.S. Patent No. 6,410,276), consensus sequence primed polymerase chain reaction (CP-PCR) (U.S. Patent No. 4,437,975), arbitrarily primed polymerase chain reaction (AP-PCR) (U.S. Patent Nos. 5, 413,909, 5,861,245) and nucleic acid based sequence amplification (NASBA). *(See,* U.S. Patent Nos. 5,409,818, 5,554,517, and 6,063,603), each of which is incorporated herein by reference). Other amplification methods that may be used include: Qbeta Replicase, described in PCT Patent Application No. PCT/US87/00880, isothermal amplification methods such as SDA, described in Walker et al. 1992, Nucleic Acids Res. 20(7): 1691-6, 1992, and rolling circle amplification, described in U.S. Pat. No. 5,648,245. Other amplification methods that may be used are described in U.S. Patent Nos. 5,242,794, 5,494,810, 4,988,617 and in U.S. Serial Number 09/854,317, each of which is incorporated herein by reference. Other amplification methods that may be used are disclosed in US Patent Application Publication No. 20030143599.

Additional methods of sample preparation and techniques for reducing the complexity of a nucleic sample are described in Dong et al., *Genome Research* 11, 1418 (2001), in U.S. Patent Number 6,361,947, 6,391,592 and U.S. Serial Nos. 09/916,135, 09/920,491 (U.S. Patent Application Publication 20030096235), 09/910,292 (U.S. Patent Application Publication 20030082543), and 10/013,598.

Methods for conducting polynucleotide hybridization assays have been well developed in the art. Hybridization assay procedures and conditions will vary depending on the application and are selected in accordance with the general binding methods known, including those referred to in: Maniatis et al. *Molecular Cloning: A Laboratory Manual* (2^{nd} Ed. Cold Spring Harbor, N.Y, 1989); Berger and Kimmel *Methods in Enzymology,* Vol. 152, *Guide to Molecular Cloning Techniques* (Academic Press, Inc., San Diego, CA, 1987); Young and Davism, *P.N.A.S,* 80: 1194 (1983). Methods and apparatus for carrying out repeated and controlled hybridization reactions have been described in U.S. Patent Nos. 5,871,928, 5,874,219, 6,045,996 and 6,386,749, 6,391,623 each of which is incorporated herein by reference

The present invention also contemplates signal detection of hybridization between ligands in certain preferred embodiments. See U.S. Patent Nos. 5,143,854, 5,578,832; 5,631,734; 5,834,758; 5,936,324; 5,981,956; 6,025,601; 6,141,096; 6,185,030; 6,201,639; 6,218,803; and 6,225,625, in U.S. Serial Number 10/389,194 and in PCT Application PCT/US99/06097 (published as WO99/47964), each of which also is hereby incorporated by reference in its entirety for all purposes.

Methods and apparatus for signal detection and processing of intensity data are disclosed in, for example, U.S. Patent Nos. 5,143,854, 5,547,839, 5,578,832, 5,631,734, 5,800,992, 5,834,758; 5,856,092, 5,902,723, 5,936,324, 5,981,956, 6,025,601, 6,090,555, 6,141,096, 6,185,030, 6,201,639; 6,218,803; and 6,225,625, in U.S. Serial Nos. 10/389,194, 60/493,495 and in PCT Application PCT/US99/06097 (published as WO99/47964), each of which also is hereby incorporated by reference in its entirety for all purposes.

The practice of the present invention may also employ conventional biology methods, software and systems. Computer software products of the invention typically include computer readable medium having computer-executable instructions for performing the logic steps of the method of the invention. Suitable computer readable medium include floppy disk, CD-ROM/DVD/DVD-ROM, hard-disk drive, flash memory, ROM/RAM, magnetic tapes and etc. The computer executable instructions may be written in a suitable computer language or combination of several languages. Basic computational biology methods are described in, for example Setubal and Meidanis et al., *Introduction to Computational Biology Methods* (PWS Publishing Company, Boston, 1997); Salzberg, Searles, Kasif, (Ed.), *Computational Methods in Molecular Biology,* (Elsevier, Amsterdam, 1998); Rashidi and Buehler, *Bioinformatics Basics: Application in Biological Science and Medicine* (CRC Press, London, 2000) and Ouelette and Bzevanis *Bioinformatics: A Practical Guide for Analysis of Gene and Proteins* (Wiley & Sons, Inc., 2^{nd} ed., 2001). See U.S. Patent Number 6,420,108.

The present invention may also make use of various computer program products and software for a variety of purposes, such as probe design, management of data, analysis, and instrument operation. See, U.S. Patent Nos. 5,593,839, 5,795,716, 5,733,729, 5,974,164, 6,066,454, 6,090,555, 6,185,561, 6,188,783, 6,223,127, 6,229,911 and 6,308,170.

Additionally, the present invention may have preferred embodiments that include methods for providing genetic information over networks such as the Internet as shown in U.S. Serial Nos. 10/197,621, 10/063,559 (United States Publication Number 20020183936), 10/065,856, 10/065,868, 10/328,818, 10/328,872, 10/423,403, and 60/482,389.

### b) Definitions

The term "array" as used herein refers to an intentionally created collection of molecules which can be prepared either synthetically or biosynthetically. The molecules in the array can be identical or different from each other. The array can assume a variety of formats*, for example,* libraries of soluble molecules; libraries of compounds tethered to resin beads, silica chips, or other solid supports.

The term "barcode" is used to refer to a unique combination of nucleic acid sequences. In a preferred embodiment the barcode includes a combination of two or more different marker molecules. Each marker molecule contains a unique tag sequence (barcode sequence) of at least 15 or at least 20 bases. The tag sequence or sequences are preferably part of a larger nucleic acid, for example a marker plasmid or within a marker fragment. The barcode may be generated by mixing two or more marker molecules in with a nucleic acid sample, for example, a genomic DNA sample from one or more individuals. The marker molecules can be added individually to the sample or they may be added in combinations of two or more.

Each marker molecule preferably comprises a stretch of 15-200 bases, more preferably 20-60 bases, or 20-40 bases, of nucleic acid tag or barcode sequence that do not naturally occur in the nucleic acid sample. For example, often the sample is genomic DNA from a human and the tag sequences are selected by comparing a candidate tag sequence to a database of known sequences to identify sequences that are not significantly homologous to a known human sequence. Preferably tag sequences are selected so that they will not cross hybridize to known human sequences under selected hybridization conditions. In preferred embodiments the tag sequences comprise 1, 2, 3, 4, 5 or more shorter tag sequences selected from a set of tag sequences. Sets of tag sequences and methods of selecting tag sequences are disclosed in U.S. Patent No. 6,458,530 and U.S. Patent Application No. 09/827,383. In general tag sequences in a set are all the same length, have TMs that are within the same temperature range, plus or minus 2 to 5 °C, and do not cross hybridize to other tags in the set or to the complement of other tags in the set or to sequences in the genome of a selected organism or organisms.

The term "barcode plasmid" refers to a construct that includes a plasmid with at least one barcode sequence insert. In preferred embodiments a barcode sequence of about 15-200 bases, more preferably 20-40 bases, or 20-60 bases, is cloned into one or more restriction sites of a plasmid. The barcode sequence is preferably cloned into the plasmid so that it can be released by digestion with a single enzyme selected from a set of enzymes to generate a restriction fragment of between 200 and 1,000 bases that contains the barcode sequence.

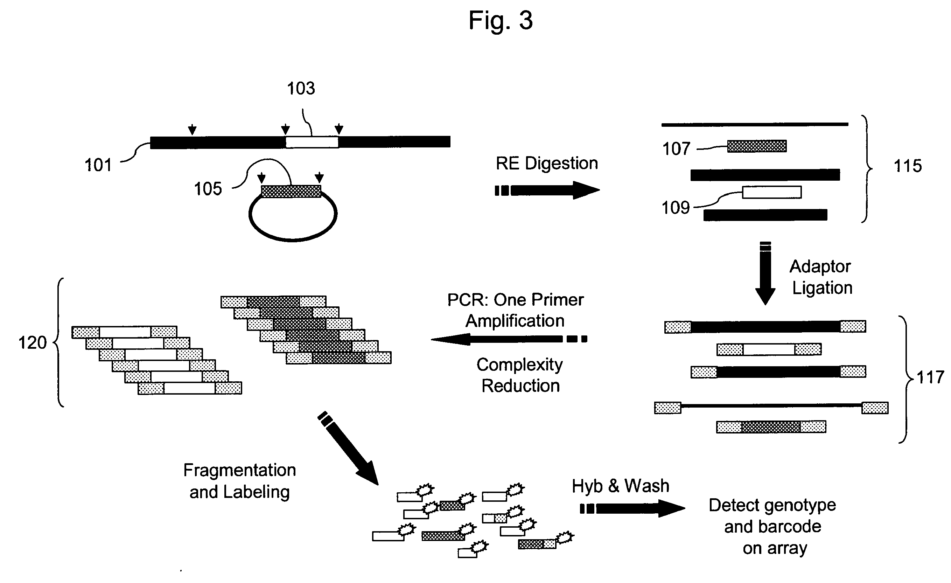

The term "barcode adaptor" refers to a nucleic acid fragment that comprises one or more barcode sequences. Barcode adaptors are shown in Figure 3. A barcode adaptor may be two synthetic oligonucleotides hybridized together to form an adaptor. The barcode adaptor preferably has at least one single stranded overhang, or "sticky end" to facilitate ligation. The barcode adaptor may also comprise other sequences, such as priming sites, recognition sites for restriction enzymes or a promoter sites for an RNA polymerase. One or more barcode plasmids or barcode adaptors may be added to the nucleic acid sample to mark the sample with a barcode. The barcode may be a combination of one or more barcode plasmids and one or more barcode adaptors.

The term "biomonomer" as used herein refers to a single unit of biopolymer, which can be linked with the same or other biomonomers to form a biopolymer (for example, a single amino acid or nucleotide with two linking groups one or both of which may have removable protecting groups) or a single unit which is not part of a biopolymer. Thus, for example, a nucleotide is a biomonomer within an oligonucleotide biopolymer, and an amino acid is a biomonomer within a protein or peptide biopolymer; avidin, biotin, antibodies, antibody fragments, etc., for example, are also biomonomers.

The term "biopolymer" (sometimes referred to as "biological polymer") as used herein is intended to mean repeating units of biological or chemical moieties. Representative biopolymers include, but are not limited to, nucleic acids, oligonucleotides, amino acids, proteins, peptides, hormones, oligosaccharides, lipids, glycolipids, lipopolysaccharides, phospholipids, synthetic analogues of the foregoing, including, but not limited to, inverted nucleotides, peptide nucleic acids, Meta-DNA, and combinations of the above.

The term "biopolymer synthesis" as used herein is intended to encompass the synthetic production, both organic and inorganic, of a biopolymer. Related to a bioploymer is a "biomonomer".

The term "combinatorial synthesis strategy" as used herein refers to an ordered strategy for parallel synthesis of diverse polymer sequences by sequential addition of reagents which may be represented by a reactant matrix and a switch matrix, the product of which is a product matrix. A reactant matrix is a 1 column by m row matrix of the building blocks to be added. The switch matrix is all or a subset of the binary numbers, preferably ordered, between 1 and m arranged in columns. A "binary strategy" is one in which at least two successive steps illuminate a portion, often half, of a region of interest on the substrate. In a binary synthesis strategy, all possible compounds which can be formed from an ordered set of reactants are formed. In most preferred embodiments, binary synthesis refers to a synthesis strategy which also factors a previous addition step. For example, a strategy in which a switch matrix for a masking strategy halves regions that were previously illuminated, illuminating about half of the previously illuminated region and protecting the remaining half (while also protecting about half of previously protected regions and illuminating about half of previously protected regions). It will be recognized that binary rounds may be interspersed with non-binary rounds and that only a portion of a substrate may be subjected to a binary scheme. A combinatorial "masking" strategy is a synthesis which uses light or other spatially selective deprotecting or activating agents to remove protecting groups from materials for addition of other materials such as amino acids.

The term "complementary" as used herein refers to the hybridization or base pairing between nucleotides or nucleic acids, such as, for instance, between the two strands of a double stranded DNA molecule or between an oligonucleotide primer and a primer binding site on a single stranded nucleic acid to be sequenced or amplified. Complementary nucleotides are, generally, A and T (or A and U), or C and G. Two single stranded RNA or DNA molecules are said to be complementary when the nucleotides of one strand, optimally aligned and compared and with appropriate nucleotide insertions or deletions, pair with at least about 80% of the nucleotides of the other strand, usually at least about 90% to 95%, and more preferably from about 98 to 100%. Alternatively, complementarity exists when an RNA or DNA strand will hybridize under selective hybridization conditions to its complement. Typically, selective hybridization will occur when there is at least about 65% complementary over a stretch of at least 14 to 25 nucleotides, preferably at least about 75%, more preferably at least about 90% complementary. See, M. Kanehisa Nucleic Acids Res. 12:203 (1984), incorporated herein by reference.

The term "effective amount" as used herein refers to an amount sufficient to induce a desired result.

The term "genome" as used herein is all the genetic material in the chromosomes of an organism. DNA derived from the genetic material in the chromosomes of a particular organism is genomic DNA. A genomic library is a collection of clones made from a set of randomly generated overlapping DNA fragments representing the entire genome of an organism.

The term "genotype" as used herein refers to the genetic information an individual carries at one or more positions in the genome. A genotype may refer to the information present at a single polymorphism, for example, a single SNP. For example, if a SNP is biallelic and can be either an A or a C then if an individual is homozygous for A at that position the genotype of the SNP is homozygous A or AA. Genotype may also refer to the information present at a plurality of polymorphic positions.

The term "hybridization" as used herein refers to the process in which two single-stranded polynucleotides bind non-covalently to form a stable double-stranded polynucleotide; triple-stranded hybridization is also theoretically possible. The resulting (usually) double-stranded polynucleotide is a "hybrid." The proportion of the population of polynucleotides that forms stable hybrids is referred to herein as the "degree of hybridization." Hybridizations are usually performed under stringent conditions, for example, at a salt concentration of no more than about 1 M and a temperature of at least 25°C. For example, conditions of 5X SSPE (750 mM NaCl, 50 mM NaPhosphate, 5 mM EDTA, pH 7.4) and a temperature of 25-30°C are suitable for allele-specific probe hybridizations or conditions of 100 mM MES, 1 M [Na⁺], 20 mM EDTA, 0.01% Tween-20 and a temperature of 30-50°C, preferably at about 45-50°C. Hybridizations may be performed in the presence of agents such as herring sperm DNA at about 0.1 mg/ml, acetylated BSA at about 0.5 mg/ml. As other factors may affect the stringency of hybridization, including base composition and length of the complementary strands, presence of organic solvents and extent of base mismatching, the combination of parameters is more important than the absolute measure of any one alone. Hybridization conditions suitable for microarrays are described in the Gene Expression Technical Manual, 2004 and the GeneChip Mapping Assay Manual, 2004.

The term "hybridization probes" as used herein are oligonucleotides capable of binding in a base-specific manner to a complementary strand of nucleic acid. Such probes include peptide nucleic acids, as described in Nielsen et al., *Science* 254, 1497-1500 (1991), LNAs, as described in Koshkin et al. *Tetrahedron* 54:3607-3630, 1998, and US Patent No. 6,268,490 and other nucleic acid analogs and nucleic acid mimetics.

The term "hybridizing specifically to" as used herein refers to the binding, duplexing, or hybridizing of a molecule only to a particular nucleotide sequence or sequences under stringent conditions when that sequence is present in a complex mixture *(for example,* total cellular) DNA or RNA.

The term "initiation biomonomer" or "initiator biomonomer" as used herein is meant to indicate the first biomonomer which is covalently attached via reactive nucleophiles to the surface of the polymer, or the first biomonomer which is attached to a linker or spacer arm attached to the polymer, the linker or spacer arm being attached to the polymer via reactive nucleophiles.

The term "isolated nucleic acid" as used herein means an object species invention that is the predominant species present (*i.e*., on a molar basis it is more abundant than any other individual species in the composition). Preferably, an isolated nucleic acid comprises at least about 50, 80 or 90% (on a molar basis) of all macromolecular species present. Most preferably, the object species is purified to essential homogeneity (contaminant species cannot be detected in the composition by conventional detection methods).

The term "mixed population" (sometimes referred to as "complex population") as used herein refers to any sample containing both desired and undesired nucleic acids. As a non-limiting example, a complex population of nucleic acids may be total genomic DNA, total genomic RNA or a combination thereof. Moreover, a complex population of nucleic acids may have been enriched for a given population but include other undesirable populations. For example, a complex population of nucleic acids may be a sample which has been enriched for desired messenger RNA (mRNA) sequences but still includes some undesired ribosomal RNA sequences (rRNA).

The term "monomer" as used herein refers to any member of the set of molecules that can be joined together to form an oligomer or polymer. The set of monomers useful in the present invention includes, but is not restricted to, for the example of (poly)peptide synthesis, the set of L-amino acids, D-amino acids, or synthetic amino acids. As used herein, "monomer" refers to any member of a basis set for synthesis of an oligomer. For example, dimers of L-amino acids form a basis set of 400 "monomers" for synthesis of polypeptides. Different basis sets of monomers may be used at successive steps in the synthesis of a polymer. The term "monomer" also refers to a chemical subunit that can be combined with a different chemical subunit to form a compound larger than either subunit alone.

The term "nucleic acid library" (sometimes referred to as "array") as used herein refers to an intentionally created collection of nucleic acids which can be prepared either synthetically or biosynthetically and screened for biological activity in a variety of different formats (for example, libraries of soluble molecules; and libraries of oligos tethered to resin beads, silica chips, or other solid supports). Additionally, the term "array" is meant to include those libraries of nucleic acids which can be prepared by spotting nucleic acids of essentially any length (for example, from 1 to about 1000 nucleotide monomers in length) onto a substrate. The term "nucleic acid" as used herein refers to a polymeric form of nucleotides of any length, either ribonucleotides, deoxyribonucleotides or peptide nucleic acids (PNAs), that comprise purine and pyrimidine bases, or other natural, chemically or biochemically modified, non-natural, or derivatized nucleotide bases. The backbone of the polynucleotide can comprise sugars and phosphate groups, as may typically be found in RNA or DNA, or modified or substituted sugar or phosphate groups. A polynucleotide may comprise modified nucleotides, such as methylated nucleotides and nucleotide analogs. The sequence of nucleotides may be interrupted by non-nucleotide components. Thus the terms nucleoside, nucleotide, deoxynucleoside and deoxynucleotide generally include analogs such as those described herein. These analogs are those molecules having some structural features in common with a naturally occurring nucleoside or nucleotide such that when incorporated into a nucleic acid or oligonucleoside sequence, they allow hybridization with a naturally occurring nucleic acid sequence in solution. Typically, these analogs are derived from naturally occurring nucleosides and nucleotides by replacing and/or modifying the base, the ribose or the phosphodiester moiety. The changes can be tailor made to stabilize or destabilize hybrid formation or enhance the specificity of hybridization with a complementary nucleic acid sequence as desired.

The term "nucleic acids" as used herein may include any polymer or oligomer of pyrimidine and purine bases, preferably cytosine, thymine, and uracil, and adenine and guanine, respectively. *See* Albert L. Lehninger, PRINCIPLES OF BIOCHEMISTRY, at 793-800 (Worth Pub. 1982). Indeed, the present invention contemplates any deoxyribonucleotide, ribonucleotide or peptide nucleic acid component, and any chemical variants thereof, such as methylated, hydroxymethylated or glucosylated forms of these bases, and the like. The polymers or oligomers may be heterogeneous or homogeneous in composition, and may be isolated from naturally-occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states.

The term "oligonucleotide" (sometimes referred to as "polynucleotide") as used herein refers to a nucleic acid ranging from at least 2, preferable at least 8, and more preferably at least 20 nucleotides in length or a compound that specifically hybridizes to a polynucleotide. Polynucleotides of the present invention include sequences of deoxyribonucleic acid (DNA) or ribonucleic acid (RNA) which may be isolated from natural sources, recombinantly produced or artificially synthesized and mimetics thereof. A further example of a polynucleotide of the present invention may be peptide nucleic acid (PNA). The invention also encompasses situations in which there is a nontraditional base pairing such as Hoogsteen base pairing which has been identified in certain tRNA molecules and postulated to exist in a triple helix. "Polynucleotide" and "oligonucleotide" are used interchangeably in this application.

The term "primer" as used herein refers to a single-stranded oligonucleotide capable of acting as a point of initiation for template-directed DNA synthesis under suitable conditions for example, buffer and temperature, in the presence of four different nucleoside triphosphates and an agent for polymerization, such as, for example, DNA or RNA polymerase or reverse transcriptase. The length of the primer, in any given case, depends on, for example, the intended use of the primer, and generally ranges from 15 to 30 nucleotides. Short primer molecules generally require cooler temperatures to form sufficiently stable hybrid complexes with the template. A primer need not reflect the exact sequence of the template but must be sufficiently complementary to hybridize with such template. The primer site is the area of the template to which a primer hybridizes. The primer pair is a set of primers including a 5' upstream primer that hybridizes with the 5' end of the sequence to be amplified and a 3' downstream primer that hybridizes with the complement of the 3' end of the sequence to be amplified.

The term "probe" as used herein refers to a surface-immobilized molecule that can be recognized by a particular target. See U.S. Patent Number 6,582,908 for an example of arrays having all possible combinations of probes with 10, 12, and more bases. Examples of probes that can be investigated by this invention include, but are not restricted to, agonists and antagonists for cell membrane receptors, toxins and venoms, viral epitopes, hormones (for example, opioid peptides, steroids, etc.), hormone receptors, peptides, enzymes, enzyme substrates, cofactors, drugs, lectins, sugars, oligonucleotides, nucleic acids, oligosaccharides, proteins, and monoclonal antibodies.

The term "solid support", "support", and "substrate" as used herein are used interchangeably and refer to a material or group of materials having a rigid or semi-rigid surface or surfaces. In many embodiments, at least one surface of the solid support will be substantially flat, although in some embodiments it may be desirable to physically separate synthesis regions for different compounds with, for example, wells, raised regions, pins, etched trenches, or the like. According to other embodiments, the solid support(s) will take the form of beads, resins, gels, microspheres, or other geometric configurations. See U.S. Patent Number 5,744,305 for exemplary substrates.

The term "target" as used herein refers to a molecule that has an affinity for a given probe. Targets may be naturally-occurring or man-made molecules. Also, they can be employed in their unaltered state or as aggregates with other species. Targets may be attached, covalently or noncovalently, to a binding member, either directly or via a specific binding substance. Examples of targets which can be employed by this invention include, but are not restricted to, antibodies, cell membrane receptors, monoclonal antibodies and antisera reactive with specific antigenic determinants (such as on viruses, cells or other materials), drugs, oligonucleotides, nucleic acids, peptides, cofactors, lectins, sugars, polysaccharides, cells, cellular membranes, and organelles. Targets are sometimes referred to in the art as anti-probes. As the term target is used herein, no difference in meaning is intended. A "Probe Target Pair" is formed when two macromolecules have combined through molecular recognition to form a complex.

### Molecular barcodes for internally marking and tracking samples

Many methods of genetic analysis require analysis of large numbers of different samples. Each sample may be derived from a different individual or a different source, and keeping track of sample identity is frequently essential to analysis of results. Samples may become contaminated by other samples, the identity of a sample may be lost, or a sample may become misidentified. Methods of integrally marking a sample in a detectable manner are disclosed. In a preferred embodiment the sample is marked by the addition of at least one amplifiable tag sequence and detection of the sequence takes place in parallel with the analysis of the biological samples. Plasmids carrying tag sequences and examples of tag sequences that may be used in the presently disclosed methods have been disclosed in U.S. Patent Publication No. 20040175719, U.S. Patent Application No. 09/827,383 and U.S. Patent No. 6,458,530. Methods of marking samples have been disclosed in, for example, US Patent Pub. No. 20040166520 and U.S. Patent Nos. 6,544,739, 5,643,728 and 5,451,505.

Technological advances in recent years have provided researchers and clinicians with the ability to process large numbers of biological samples for analysis of genotype. These methods may be used, for example, to diagnose disease or risk of disease, or to identify associations between a phenotype and a genetic region. Genotyping methods may also be used for forensic purposes and for paternity analysis. For most of these genotyping applications it is important that the sample be properly identified as to source of origin and that during subsequent manipulation steps the sample can be correctly identified. Marking the container in which a sample is placed has been one method of marking but sample mix ups, such as errors in labeling and cross-contamination between samples do occur and can be difficult to detect without a marking method that is integrated within the sample itself. The presently disclosed methods provide mechanisms to mark a sample so that sample identities can be checked to prevent misidentification of a sample and to detect cross contamination between samples.

The disclosed methods may be used, for example, for marking and tracking biological samples with known combinations of marker molecules carrying tag sequences. The methods may be particularly useful for tracking samples in high throughput assays, for example, when samples are treated or stored in multiwell plates. In one embodiment different amounts of a plurality of control nucleic acids are added to each of a plurality of genomic samples. The spike in controls may be amplified along with the sample and analyzed in parallel with analysis of the sample, for example, in a gene expression or genotyping analysis method. In a preferred embodiment the analysis includes a step of hybridization to an array of probes. Samples can be analyzed to detect the marker molecules by other methods such as PCR. The control nucleic acids in a preferred embodiment are combinatorial sets of plasmids where each plasmid contains a different barcode sequence, for example a 40 base pair sequence. In a preferred embodiment the barcode sequence may be a tag sequence of length of at least 20 bases.

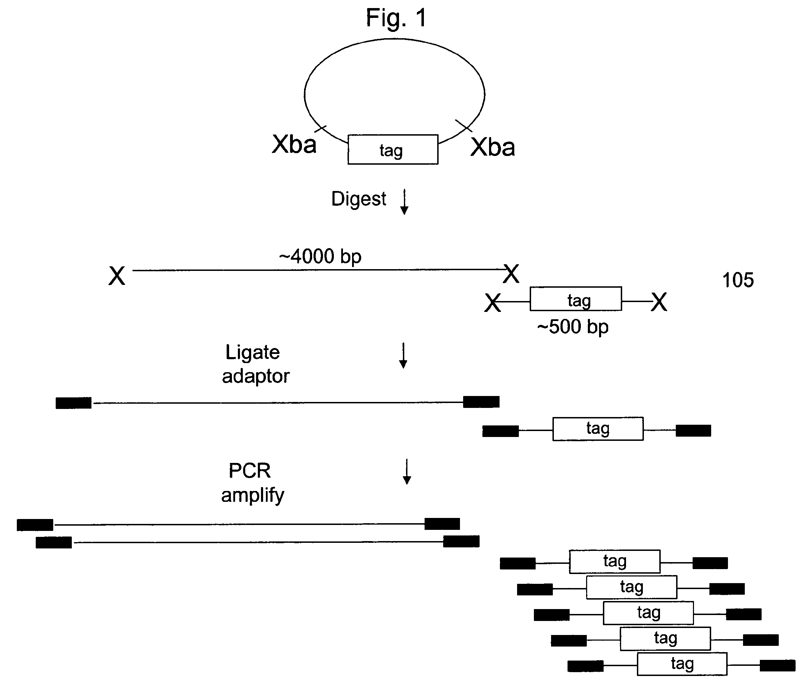

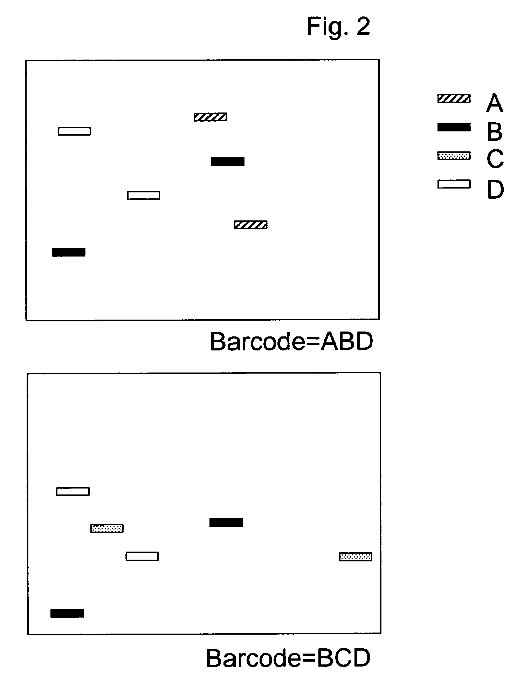

In Figure 1 a barcode plasmid marker molecule is shown. The barcode sequence is a tag sequence that is flanked by Xba I sites. When the plasmid or a sample containing the plasmid is digested with Xba I, two fragments result. The larger fragment shown is about 4 Kb and the smaller, which contains the tag sequence that may be used as a component of a barcode, is about 500 base pairs. After adaptor ligation the fragment containing the tag sequence can be efficiently amplified by PCR using a primer to the adaptor sequence. The larger fragment is inefficiently amplified. Figure 2 shows a schematic of hybridization patterns resulting from the presence of different barcodes in different samples. In the top panel tag sequences A, B and D are present and hybridization is detected at the A, B, and D probe sets but not at the C probe set. In the lower panel the hybridization pattern shows that tags B, C and D are present but not A. The probe sets for the tag sequences are present at different locations of the array and are present in duplicate on the array.

In one aspect (Fig. 3) the barcode is identified by the same process that is used to analyze sequences of interest in the sample. The sample containing genomic DNA (101) with sequence of interest (103) has been marked with a marker plasmid (105) carrying a tag sequence. Restriction sites (indicated by arrows) for a selected enzyme, for example, Sty I, flank the tag sequence and the sequence of interest. Upon digestion with the restriction enzyme a variety of restriction fragments (115), including a fragment that includes the tag sequence (107) and a fragment that includes the sequence of interest (109), are generated. An adaptor is ligated to the restriction fragments to generate adaptor-ligated fragments (117). The adaptor ligated fragments are amplified using a primer complementary to the adaptor. This reduces the complexity of the sample because only fragments that are in a selected size range (about 200 to 2000 base pairs) are efficiently amplified. The amplified fragments are subjected to an additional fragmentation step followed by labeling. The labeled fragments are then hybridized to an array. The array has probes to analyze the sequence of interest and to detect the tag sequence. In preferred aspects the sequence of interest includes a SNP and the array includes probes to determine the allele or alleles present at that SNP. In preferred aspects the array is a genotyping array such as the GENECHIP® Mapping 100K set (Part Numbers 900517 and 900523) or GENECHIP® Mapping 500K set available from Affymetrix, Inc., Santa Clara.

In some embodiments the barcode method may be used to mark biological samples prior to gene expression analysis. The marker molecules may include a polyA sequence 3' of the tag sequence and a promoter sequence for a phage polymerase, such as T3, T7 and SP6 RNA polymerase, 5' of the tag sequence. The sample may be reverse transcribed in parallel with the sample using an oligo dT primer or a random primer to make first strand cDNA. The first strand cDNA can be converted to double stranded cDNA, including a promoter for RNA polymerase, using standard methods. Many RNA copies of the tag sequence may be transcribed using RNA polymerase.

In a preferred aspect the method involves adding a unique combination of at least two different DNA tag molecules of known sequence to each of a plurality of genomic DNA samples so that a different combination of DNA tag molecules is added to each of the samples. The samples are subjected to an amplification and complexity reduction step including fragmentation with a restriction enzyme, adapter ligation and amplification using a primer complementary to the adapter. The complexity is reduced because only restriction fragments that fall within a limited size range are efficiently amplified. For example, fragments that are about 200 to 2000 base pairs are efficiently amplified and smaller or larger fragments are not amplified or are poorly amplified. The DNA tag molecules are designed so that the tags are on fragments that will be efficiently amplified during the complexity reduction and amplification step. The reduced complexity sample, including the tags, may then be analyzed by hybridization to an array of probes. The array preferably includes probes to detect each of the different tags. Different combinations of tags should result in a different hybridization pattern that is characteristic of the combination of tags included in the sample.

The methods may be used for tracking samples. Each sample is mixed with a different spiked-in set of molecules containing tag sequences so that each sample gets a barcode, which is a combination of tag sequences, that varies from the barcode in every other sample in the sample set, by at least one tag sequence.

In a preferred embodiment samples are marked by the addition of a nucleic acid barcode to the sample. The barcode may be added to the sample during isolation of the sample from the biological source. Alternatively the barcode can be added after isolation of the desired sample from the source, for example, the barcode may be added to a cell lysate, an isolated nucleic acid sample, an isolated genomic DNA sample, or an isolated RNA sample. In a preferred embodiment the sample is a biological sample in solution and a solution of each barcode marker is added so that the barcode marker is mixed into the sample. In preferred aspects the barcode marker includes two or more independent marker molecules, for example, two or more different sequence plasmids or two or more different sequence fragments. When an aliquot of the biological sample is removed it preferably contains at least one copy of each marker molecule and preferably contains a plurality of each marker molecule.

In a preferred embodiment the barcode is amplified when the nucleic acid sample is amplified. The barcode is designed so that the conditions used for amplification of the sample will result in amplification of the barcode. For example, if the sample will be amplified by reverse transcription with an oligo dT primer, the barcode may include a polyA sequence.

In a preferred embodiment the barcodes are detected by hybridization to oligonucleotide probes that are complementary to sequences within the barcode. The probes may be attached to a solid support, for example, a chip, a membrane or a bead.

Marker molecules are preferably used in combinations of 2 or more in each sample. A small number of different tag sequences may be used to generate a large number of different barcodes because the plasmids may be combined in many independent combinations. The barcodes may be detected using probes to the limited number of tag sequences. The barcode in each sample will hybridize to a different combination of the tag probes. In this way a limited number of detection probes may be used. For example, 6 different 2 letter combinations can be made from the letters A, B, C and D (AB, BC, CD, AC, AD, and BD) so 6 samples can be uniquely marked with different combinations of 4 tags. In general the formula for the number of different permutations of K objects from a set ofN objects is: N!/[K!(N-K)!]. For example, if there are 20 different marker molecules there are 190 different possible combinations of 2 marker molecules, so 190 different barcodes [20!/2!(20-2)! = (20x19)/2=190]. For example, 20 tag sequences can be used to uniquely mark each of 190 different sequences with a unique barcode. Each of the 190 barcodes can be uniquely detected using the same 20 tag sequence detection probes or probe sets. Similarly, a set of 10 marker molecules can be used to make 45 different combinations of 2 marker molecules, 120 different combinations of 3, and 210 different combinations of 4. A set of 20 different marker molecules can be used to make 1140 combinations of 3 or 4845 combinations of 4. A set of 30 different marker molecules can be used to make 435 combinations of 2, 4060 combinations of 3 and 27,405 combinations of 4. In each of these sets all possible combinations can be detected using the same limited set of probes complementary to the tag or barcode sequence in the different marker molecules. For example, the 27,405 different combinations of marker molecules that are possible when a set of 30 different marker molecules are used in combinations of 4, can be detected using 30 probe sets, where each probe set detects the tag or barcode sequence in one of the marker molecules. The probe set comprises one or more probes that are perfectly complementary to at least 20 contiguous bases of tag sequence present in the marker molecule.

The methods are particularly well suited to standard multiwell plate formats. For example 20 barcode plasmids may be used to uniquely barcode each well of a 96 well plate, having 12 columns and 8 rows of wells. The same marker sequence is added to each well of each row for each of the 8 rows, 1 plasmid in each row and one plasmid is used in each of the 12 columns. Each well will have a different combination of two of the 20 plasmids resulting in a different barcode combination for each sample.

In a preferred embodiment the sample is prepared for hybridization and analysis using the whole genome sample assay (WGSA) as described in Matsusaki et al., *Nature Methods* 1:109-111 (2004). The WGSA method amplifies a reproducible subset of fragments from genomic DNA samples. The sample is fragmented with a restriction enzyme, adaptors are ligated to the fragments and the fragments are amplified by PCR using a primer that is complementary to the adaptor. Only fragments that are within a limited size range, about 200 to 2500 base pairs, are efficiently amplified. The fragments that will be amplified can be predicted by doing an *in silico* digestion of the genome. The barcode plasmids are preferably constructed so that when the genomic sample containing the barcode plasmid or plasmids is fragmented the barcode will be present on a fragment that will be efficiently amplified, for example, a fragment that is between 200 and 2500 base pairs, a and more preferably between 300 and 1,000 base pairs.

In a preferred embodiment each of the barcode plasmids is constructed to contain a different barcode sequence region that includes 2 or more 20 base tag sequences. The plasmid is designed so that the fragment containing the barcode region is released as a fragment that will be efficiently amplified after digestion with a selected enzyme. In preferred embodiments the fragment containing the barcode region is released after digestion with Xba I, Hind III, Nsp I or Sty I. Plasmids containing barcode regions may be prepared in large batches. Small amounts of each plasmid may be used in each sample, for example, about 50 pg each marker plasmid per 250 ng genomic DNA. The presence of the barcode may also be detected by PCR. This may be used as a quality control mechanism. The probes that detect the tag sequences preferably do not cross hybridize to genomic DNA, to other tags or tag probes or to other probes of the array. The array preferably has probes or probe sets that uniquely detect each marker molecule or tag sequence. In a preferred embodiment tag sequences are selected so that each hybridizes with similar intensity to the array.

In a preferred embodiment the barcodes are used to mark samples in a genotyping assay as described in U.S. Patent Application Nos. 10/880,143 and 10/891,260 and U.S. Patent Pub. Nos. 20040067493 and 20040146890, each of which is incorporated herein by reference in its entirety. Briefly, a genomic sample is digested with a selected restriction enzyme, an adaptor comprising a universal priming site is ligated to the ends of the fragments and the adaptor-ligated fragments are amplified by PCR using the universal priming site. Only fragments that are less than about 2 kb and greater than about 200 base pairs are efficiently amplified, resulting in an enrichment of the regions of the genome that are contained within fragments that are between 200 base pairs and 2,000 base pairs following digestion with the selected enzyme. The amplified fragments are then labeled, for example, with biotin and hybridized to an array comprising allele specific probes for SNPs present within fragments that are 200 to 2000 base pairs. Each allele of each SNP may be interrogated by a plurality of probes. The barcode construct has restriction sites arranged so that when the sample is cleaved with the selected restriction enzyme the barcode region will be within a fragment that is between 400 and 800 base pairs, the adaptors will ligate to the ends of the barcode fragment and the fragment will be amplified during PCR with the universal primer. The barcode fragments will be labeled during the labeling reaction and the array comprises probes to detect the amplified barcode region.

The tags are artificial sequences selected to be absent from the genome being analyzed. The tags are selected so that they do not cross hybridize to the allele specific genotyping probes on the array. Methods of selecting and using tag sequences have been disclosed, see for example, U.S. Patent No. 6,458,530 and U.S. Patent Application Nos. 09/827,383 and 10/619,739 (U.S. Publication No. 20040175719). In preferred embodiments sequences are selected to be used an internal controls that can be spiked into a sample at known levels and detected by hybridization. Preferably the sequences are not closely related to sequences in the genome of interest and more preferably the sequences that are to be used as controls are not similar to sequences in any genome that may be analyzed. Such sequences which may be referred to as "alien" or "antigenomic" may be generated by a computer, for example randomly generated sequences, and checked against databases of available sequences, for example, the GenBank database, to eliminate sequences that may cross hybridize to probes for known sequences. See also WO2004064482. In preferred embodiments the hybridization properties of the tag sequences and tag probes are selected to behave in a manner that is similar to the naturally occurring sequences that will be analyzed. When designing probes for naturally occurring sequences the sequence itself imposes constraints on the choice of probe and as a result on the behavior of the probe. For example, in a genotyping assay using allele specific probes for each allele of a selected SNP, the probes must correspond to the region surrounding and including the SNP. These probes may not have the optimal hybridization properties. Tag probes may be selected to have hybridization properties that are similar to the probes of the array that are directed to the genome of interest.

In one embodiment the probe sets for each barcode are distributed so that they are present at different locations on the array. In one embodiment the probe set for each barcode may be present in duplicate on the array but in different locations, (Fig 2).

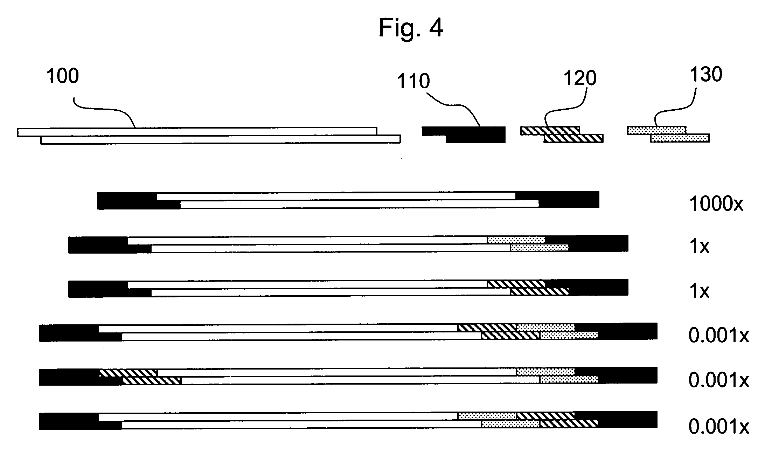

In one embodiment the barcode sequences are spiked in as barcode adaptors that may be ligated to genomic fragments (Fig 4). Genomic DNA fragments (100) are mixed with a primer adaptor sequence (110) that contains a primer site and barcode adaptors (120 and 130) that each contain a different barcode sequence. The primer adaptor may be added at amounts that are significantly higher than the barcode adaptors, for example, the primer adaptor may be added in amounts that are about 1,000 times the amount of each of the barcode adaptors. The barcode adaptors will only be ligated to a subset of the fragments. The barcode adaptors will then be amplified along with the genomic fragment that they are ligated to. The WGSA method fragments genomic DNA with a restriction enzyme and then adaptor sequences are ligated to the ends of the fragments. Barcode adaptors may be added in during the ligation step. The barcode sequences will be ligated to the genomic fragments and then to the adaptor. The barcode sequence will then be amplified along with the genomic fragment.

In a preferred embodiment about 50 pg of each marker molecule may be added to about 250 ng of genomic DNA, allowing easy identification of the barcodes following target amplification and hybridization to arrays. A standard plasmid miniprep with yield of about 10 µg provides enough plasmid for about 200,000 assays. In a preferred embodiment preparation and storage of the barcode plasmids or sequences is in an area that is free of genomic DNA samples to avoid contamination of the barcode plasmids. Barcode plasmids may be stored in a multiwell plate format. For example plasmid solutions at a concentration of 50 pg/µl may be combined in pairs using multi-channel pipets or an automated liquid handling device to yield a final concentration of 25 pg/µl of each plasmid. Care should be taken to prevent cross contamination of barcode plasmids or contamination of barcode plasmids with genomic DNA samples or amplicons.

In preferred embodiments the results of hybridization to barcode probe sets are included in a report generated by a computer after analysis of a hybridization pattern. Computer implemented methods may be used to analyze the hybridization pattern, to identify the tag sequences that are present and to compare this to a database of barcodes to identify the sample.

In one embodiment the marker molecule plasmids are added directly to the samples without linearization. In another embodiment the plasmids may be linearized or fragmented prior to being added to the sample. Preferably the plasmid is not fragmented in the region to be amplified for barcode analysis, for example, not at or between the Xba I sites if Xba I will be used for fragmentation in the subsequent detection assay.

In one embodiment a plurality of probes for the barcode sequences is screened to identify probes that perform well in a complex background. Many probes may be tested for each tag sequence and a representative set of probes may be selected. The probes may be selected to provide optimal hybridization or they may be selected on the basis of other criteria, such as detectable hybridization over a broad range of conditions and samples.

In one embodiment a set of marker molecules is provided as a kit. The kit may include a plurality of marker molecules that vary in the tag sequence they each carry. In one embodiment the marker molecules may be identical except for the variable barcode sequence. The kit may include, for example, 5-100, 5-50, 10-20, 20-50 or 50-100 different marker molecules. The marker molecules may be provided in separate containers or they may be provided in a multiwell format, for example, a microtitre plate format. Each well may contain a different marker molecule or a different known combination of marker molecules and the storage format may facilitate used of a liquid transport device that accesses multiple wells simultaneously, for example, a multi-channel pipet.

### Examples

Construction of barcode plasmids to be used as marker molecules: A set of 20 barcode plasmids was constructed. First, a vector, pFC48 (SEQ ID NO. 43), was constructed. The Xho I and Nhe I barcode-cloning sites are at positions 544-549 and 964-969. The ampicillin-resistant, pUC-based plasmid is carried in the E. coli strain FC240. The plasmid has a polyA sequences downstream, as well as the T3, SP6, and T7 transcriptional promoters, all of which may be used for gene expression analysis barcoding embodiments. To construct the barcode plasmids, phosphorylated oligo adaptors were cloned into the Xho I - Nhe I sites of the vector. The resulting 20 plasmids differ from each other only in the 40 bp tag sequence, each of which is composed of tandem GenFlex 20mer tags (see Table 1). Flanking each of the barcodes is a common Spe I restriction enzyme recognition site to allow identification of barcode clones, because the vector lacks a Spe I site. The other distinguishing feature of the plasmids is the presence of dual Xba I and Hind III restriction enzyme recognition sites, positioned such that treatment of the plasmids with either Xba I or Hind III cuts the plasmids into two pieces of approximately 500 base pairs and 4100 base pairs. The 500 base pair fragments are readily amplified by the 100K mapping assay, which includes the steps of restriction enzyme digestion with Xba I or Hind III , adaptor ligation with an adaptor containing a universal priming site and PCR amplification using a primer to the universal priming site.

SEQ ID NO. 44 shows the sequence of 100K barcode A, as an example. The Xho I and Nhe I barcode-cloning sites are at positions 544-549 and 596-601, and the 40-base barcode sequence is at positions 556-595. The other 100K barcode plasmids have the same sequence, except for the 40-base barcode.

The columns of table 1 are as follows: column 1 is the name of the barcode sequence, column 2 gives the barcode sequence, column 3 is the SEQ ID NO for the barcode sequence, column 4 is the quality control primer sequence corresponding to the barcode sequence.

### Using barcode plasmids in multiwell plates

For every 250 ng of genomic DNA (5 µL), add 2 µL of solution from a well of the barcode plate; thus the genomic sample is now irreversibly barcoded with 50 pg of each of the two barcode plasmids in that well. Subject the sample to genotyping using the Affymetrix 100K assay as described in the 100K Mapping Assay Manual, available from Affymetrix, Santa Clara. The barcode sequences are amplified and labeled along with the genomic restriction fragments. The 100K Mapping Arrays have 8 probe pairs (perfect match and mismatch) for each of the 20 barcodes. These probes were chosen to flank the central position of the 40 bp sequence at regular intervals; some of the barcodes have only antisense probes, and some have sense and antisense, as indicated in the 100K library files. No screening or probe selection was performed, so there is variation in probe intensity within a probe set as well as between probe sets.

Hybridization results for the barcodes have been measured by two methods, both developed based upon calculation of the median intensity of the perfect match probes. First, the GDAS (GeneChip DNA Analysis Software, available from Affymetrix, Inc.) report can be configured to show presence/absence of the barcode based upon intensities above a certain threshold. A safe threshold would be 5000 PM median intensity, which would allow a correct present/absent call in every experiment done to date. The advantage of this GDAS threshold report method is that it is convenient for the user; however, it gives a present/absent answer and does not readily allow for the detection of trace cross-contamination. A second output method is to report the actual PM median intensity. This can be done, for example using a special file in the GDAS folder. The advantage to this second method is that it allows the user more control over the barcode results, including the ability to detect cross-contamination from one sample to another. In preferred aspects the methods are capable of detecting cross contamination at very low levels of contamination, for example, 0.4 to 2%, 2-5%, or 5-10% contamination. For example, if a contaminated sample is 95% a first sample and 5% a second sample the first sample is contaminated by the second at 5%. Higher levels of contamination, greater than 10% may also be detected. The two methods may be combined by having the computer system report both the actual barcode intensities as well as a present/absent call, based upon user-tunable thresholds.

### Testing Barcodes

For each 100K barcode a unique, barcode-specific PCR primer was designed and tested. This quality control PCR permits a quick, sensitive check of a sample to determine which barcodes are present. For example, if the barcodes detected on the array differ from the expected barcodes, the researcher could use a small aliquot of the original archived, unamplified, barcoded sample as a PCR template for the expected and observed barcodes. The amount of barcode present in 1 ng of archived, barcoded genomic sample is sufficient template in a standard PCR to give a clear present/absent signal which may be detected as a band on a gel.

The quality control primers are given in table 1. All are designed to work with the common primer 236m13f, the sequence of which is: aacgccagggttttcccagt (SEQ ID NO. 21). Standard PCR conditions, with an annealing temperature of 55°C and extension times of 30 sec. have been used. Primers 236m13f and 235m13r (sequence: caggaaacagctatgaccatg) (SEQ ID NO. 22) may be used in a positive control amplification reaction to amplify a 753 bp product from each of the barcode plasmids. Likewise, the same PCRs can be done to test manufactured barcode preparations for the expected barcodes. In this case a sampling of templates/primers may be used.

### 30 Barcode Plasmids for 500K Mapping Assay

To accommodate the restriction sites used in the 500K mapping assay, as well as to provide for future possible enzyme fractions, the vector pFC51 was created. This vector has dual restriction enzyme recognition sites for the following 14 enzymes: Xba I, Hind III, Sty I, Nsp I, BsaJ I, Tsp45 I, Apo I, Sau3A I, HinF I, Tse I, Sau96 I, Mse I, BssK I, and PspG I. A fifteenth enzyme fraction is the enzyme pair Msp I - Ase I. An approximately 1750 base pair human genomic fragment separates the Xho I - Nhe I sites to facilitate cloning by allowing better differentiation between uncut, single-cut, and double-cut (desired) vector. The vector used to clone the 500K barcodes, pFC51 (SEQ ID NO. 45), is an ampicillin-resistant, pUC-based plasmid. The ~1750 n's indicate a random human Xho-Nhe genomic fragment used as a stuffer to aid in cloning by allowing better differentiation between uncut, single-cut, and double-cut (desired) vector. The rest of the sequence between the BamH I and EcoR I sites consists of synthetic, GenFlex Tag-derived sequence and was synthesized. There is a polyA downstream sequence, as well as T3, SP6, and T7 transcriptional promoters. The Xho I and Nhe I cloning sites are at positions 422-427 2162-2167. The vector pFC51, is carried in the strain FC243.

The final 500K barcode plasmids were constructed by ligating phosphorylated oligo adaptors encoding 40-bp tandem Tag sequences into the Xho/Nhe-digested pFC51 vector. All 30 clones were sequence-verified, and stored as glycerol stocks. Ten of the 500K clones contain the same barcodes as the corresponding 100K clones, (A, C, D, E, H, I, M, N, R, and S) but the other ten 100K clones were not suitable for 500K due to the presence of restriction sites (Sau3A I, HinF I, etc.) in the barcodes. The other twenty 500K barcodes are named 2.01 through 2.20. These plasmids should function for 10K, 100K, or 500K assays, as well as in future assays that utilize the above-named enzymes. As with the 100K clones, QC primers were designed; see Table 2 for the QC primer sequences. The sequence of 500K barcode plasmid 2.01 (SEQ ID NO. 46) is provided as an example. The Xho I barcode cloning site is at positions 422-427 and the Nhe I cloning site is at positions 474-480. The 40-base barcode sequence is at positions 434-473. The remaining 29 500K barcode plasmids have the same sequence, except for the 40-base barcode.

Because there are thirty 500K clones, it is straightforward to make more than 96 barcode combinations. One way to proceed would be to use 20 barcode plasmids to make 96 pairs as described above and make many replica plates of those 96 pairs. To one set of plates add a 21^{st} plasmid to every well for a total of 3 barcode plasmids per well; to a second set of plates add a 22^{nd} plasmid to every well; continuing this way would create 960 different combinations of 3 plasmids per well. Each well would be distinct, and each plate could be traced on the basis of barcodes 21-30. Additional plates could be created either by adding more combinations per well or by making additional barcodes.

The columns of table 2 are as follows: column 1 is the name of the barcode sequence, column 2 gives the barcode sequence, column 3 is the SEQ ID NO for the barcode sequence, column 4 is the quality control primer sequence corresponding to the barcode sequence.

### CONCLUSION

It is to be understood that the above description is intended to be illustrative and not restrictive. Many variations of the invention will be apparent to those of skill in the art upon reviewing the above description. The scope of the invention should be determined with reference to the appended claims, along with the full scope of equivalents to which such claims are entitled. All cited references, including patent and non-patent literature, are incorporated herewith by reference in their entireties for all purposes.

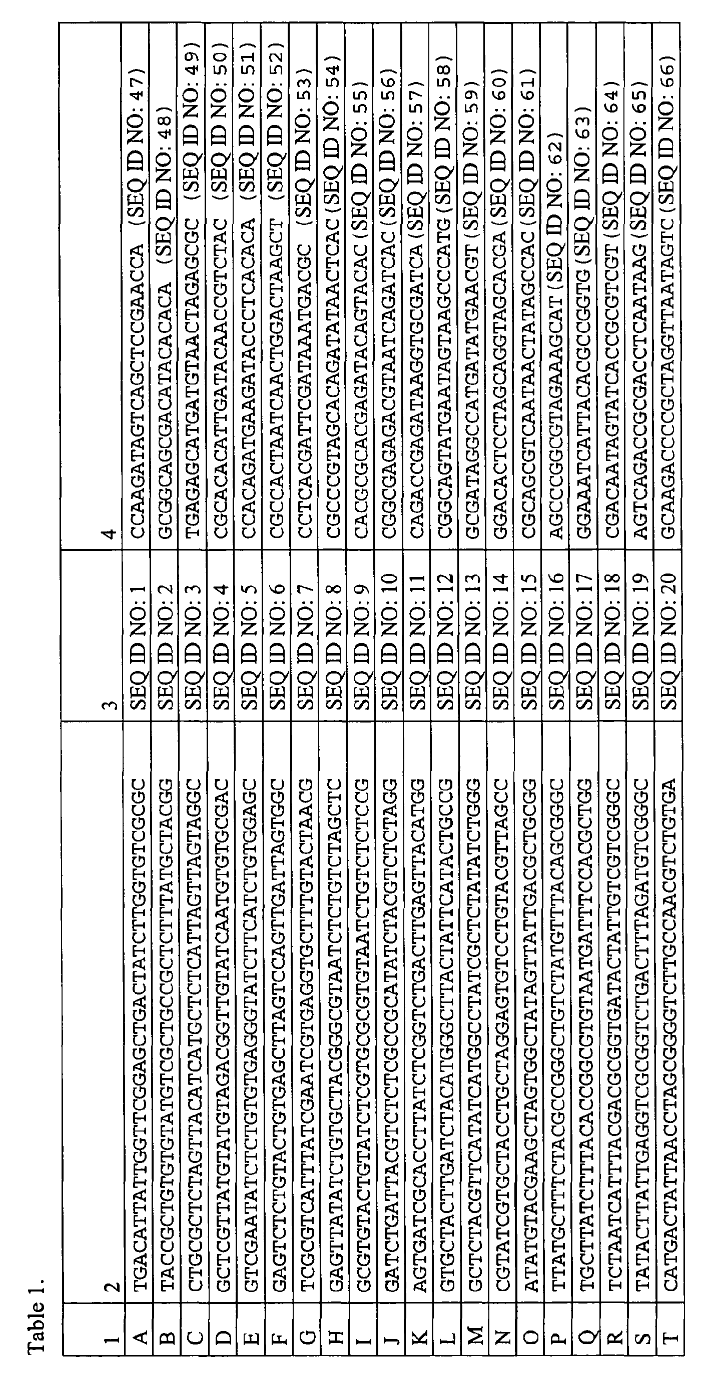

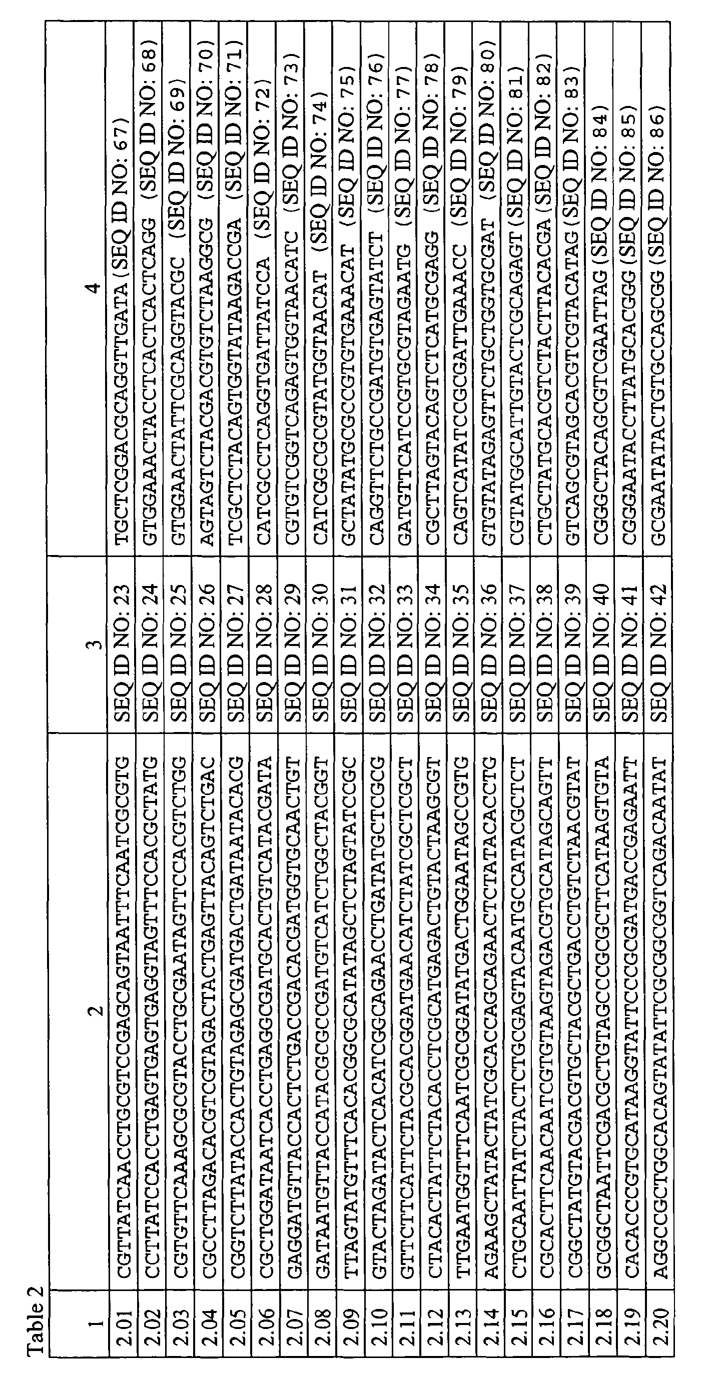

## Claims

1. A method of marking a plurality of barcoded biological samples, each comprising a different detectable barcode, said method comprising:
obtaining a plurality of different nucleic acid marker molecules, where each marker molecule comprises a different nucleic acid tag sequence;
obtaining a plurality of biological samples;
adding an aliquot of each of at least 2 of the marker molecules to each of the biological samples to generate a plurality of barcoded biological samples, wherein each of the barcoded biological samples in the plurality comprises a different combination of marker molecules.

2. The method of claim 1 wherein each different marker molecule is a barcode plasmid, wherein each barcode plasmid comprises a vector sequence and a marker insert comprising a tag sequence of at least 40 base pairs.

3. The method of claim 2 wherein the vector sequence is selected from a subsequence of at least 4,000 contiguous bases of SEQ ID NO. 43 or at least 3,500 contiguous bases of SEQ ID NO. 45.

4. The method of claim 1 wherein a detectable barcode comprises a combination of tag sequences wherein each tag sequence is detectable by hybridization to a plurality of probes.

5. The method of claim 1 wherein a tag sequence comprises between 10 and 100 contiguous bases that are not found in the human genome.

6. The method of claim 1 wherein a tag sequence comprises between 10 and 100 contiguous bases that are not found in the human, mouse, yeast, or rat genomes.

7. The method of claim 1 wherein each tag sequence comprises between 10 and 100 contiguous bases not found in available databases of naturally occurring genomic sequence.

8. The method of claim 1 wherein each of the nucleic acid marker molecules comprises a double stranded region comprising two priming sites flanking a tag sequence, wherein the tag sequence is between 20 and 200 bases.

9. A method of identifying a biological sample marked with a detectable barcode marker, according to the method of claim 1, comprising:
fragmenting the biological sample with a restriction enzyme to generate restriction fragments;
ligating an adaptor to the restriction fragments to generate adaptor-ligated fragments;
amplifying the adaptor-ligated fragments using a primer that is complementary to the adaptor;
labeling the amplified fragments with a detectable label;
hybridizing the labeled fragments to an array of probes wherein the array comprises probes that are complementary to different tag sequences in the plurality of marker molecules;
analyzing the hybridization pattern to identify which tag sequences are present in the sample;
determining the barcode present in the sample, wherein the barcode is the combination of tag sequences that are present in the sample; and
determining the identity of the sample from the barcode.

10. The method of claim 9 wherein the array further comprises a plurality of allele specific genotyping probes and the hybridization pattern is further analyzed to determine the genotype of a plurality of single nucleotide polymorphisms.

11. The method of claim 10 wherein the plurality of single nucleotide polymorphisms comprises more than 10,000 human single nucleotide polymorphisms.

12. The method of claim 10 wherein the plurality of single nucleotide polymorphisms comprises more than 500,000 human single nucleotide polymorphisms.

13. The method of claim 9 wherein the barcode comprises 2 different tag sequences.

14. The method of claim 9 wherein the barcode comprises 3 different tag sequences.

15. A method of marking each sample in a plurality of biological samples so that each sample is marked with a detectable barcode marker that is different from the barcode marker of each of the other samples in the plurality, said method comprising:
putting an aliquot of each sample in a different well of a multi-well plate comprising 12 columns and 8 rows;
obtaining 12 different first marker molecules and 8 different second marker molecules;
putting an aliquot of one of the first marker molecules into each well of each column so that each column has a different first marker molecules and all wells in a column have the same first marker molecule; and,
putting an aliquot of one of the second marker molecules into each well of each row so that each row has a different second marker molecules and all wells in a row have the same second marker molecule.

16. The method of claim 15 wherein each different first marker molecule and each different second marker molecule contains a different tag sequence.

17. The method of claim 16 wherein each different tag sequence is flanked by a first and second restriction site for a first restriction enzyme.

18. A method for marking a plurality of X genomic samples using Y different independent marker molecules, where Y is less than X, comprising:
obtaining a plurality of X genomic DNA samples;
adding an aliquot of each of two of said Y different independent marker molecules to each of the X genomic DNA samples so that no two of the X genomic DNA samples has the same combination of independent marker molecules added.

19. The method of claim 18 wherein each independent marker molecule comprises a plasmid vector backbone portion and an insert portion wherein the insert portion comprises at least one 20 base pair tag sequence.

20. The method of claim 19 wherein each independent marker molecule comprises a gene that confers a selectable phenotype.

21. The method of claim 18 wherein each independent marker molecule has at least two restriction sites for a restriction enzyme, wherein digestion of the marker sequence with the restriction enzyme generates a restriction fragment that comprises the tag sequence and is between 200 and 1000 base pairs.

22. The method of claim 18 wherein X is greater than 90 and Y is between 10 and 30.

23. A method of detecting contamination of a first sample with a second sample, wherein the first sample is marked with a first barcode and the second sample is marked with a second barcode, wherein a barcode comprises a known combination of 2 to 5 tag sequences and said first barcode and said second barcode are different:
fragmenting the first sample with a restriction enzyme to generate restriction fragments;
ligating an adaptor to the restriction fragments to generate adaptor-ligated fragments;
amplifying the adaptor-ligated fragments by polymerase chain reaction using a primer complementary to the adaptor to generate amplified fragments;
labeling the amplified fragments;
generating a hybridization pattern for said first sample by hybridizing the labeled fragments to an array of probes comprising probes complementary to said tag sequences;
analyzing the hybridization pattern to determine which tag sequences are present in said first sample; and,
determining that said first sample is contaminated with said second sample if the barcode of the second sample is detected in the first sample.

24. The method of claim 23 wherein the restriction enzyme is selected from the group consisting of Xba I, Sty I, Nsp I, Hind III and Eco RI.

25. The method of claim 23 wherein the array of probes comprises at least 10,000 different probes each present in a different feature of the array.

26. The method of claim 25 wherein the array is a genotyping array, comprising allele specific probes complementary to known human single nucleotide polymorphisms.

27. A method of determining the genotype of a sample at a plurality of single nucleotide polymorphisms and the identity of the sample, comprising:
marking the sample to be analyzed with a barcode to generate a marked sample, wherein the barcode comprises a known combination of marker molecules each carrying a detectable tag sequence;
fragmenting an aliquot of the marked sample with a restriction enzyme to generate restriction fragments;
ligating adaptors to the restriction fragments to generate adaptor-ligated fragments;
amplifying the adaptor-ligated fragments;
labeling the amplified fragments and hybridizing the labeled fragments to an array, wherein the array comprises genotyping probes and probes complementary to tag sequences to generate a hybridization pattern; and,
analyzing the hybridization pattern to determine the genotype of the sample at said plurality of single nucleotide polymorphisms and to determine the barcode.

28. The method of claim 27 wherein the known combination of marker molecules comprises 2 different marker molecules.

29. The method of claim 27 wherein the known combination of marker molecules comprises 3 different marker molecules.

30. The method of claim 27 wherein the known combination of marker molecules comprises 4 different marker molecules.

31. A kit comprising a plurality of at least 10 different nucleic acid marker molecules wherein each marker molecule is physically separated from every other marker molecule and each comprises a different tag sequence.

32. The kit of claim 31 comprising 10 to 20 different marker molecules.

33. The kit of claim 31 comprising 20 to 50 different marker molecules.

34. The kit of claim 31 wherein each different marker molecule comprises a tag sequence selected from SEQ ID NOs. 1-20 and 23-42 cloned into one or more restriction sites of SEQ ID NO. 43 or SEQ ID NO. 45.

35. The kit of claim 31 wherein each different marker molecule comprises a fragment comprising a different tag sequence wherein the fragment is cloned into the XhoI and NheI sites of SEQ ID NO. 43 or the XhoI and NheI sites of SEQ ID NO. 45.

36. A kit comprising a plurality of at least 20 different nucleic acid marker molecules wherein the marker molecules are provided in a multiwell container, so that different combinations of at least two marker molecules are present in each of a plurality of wells.

37. The kit of claim 36 wherein the multiwell container is a multiwell plate.

38. The kit of claim 36 wherein the multiwell plate comprises 96 or 384 wells and each well contains a different combination of marker molecules.

39. A kit comprising a plurality of barcode plasmids wherein each barcode plasmid comprises a different tag sequence, wherein cleavage of each barcode plasmid in the plurality with a selected restriction fragment releases the tag sequence on a restriction fragment that is between 200 and 2000 base pairs in length.

40. The kit of claim 39 wherein the restriction fragment is between 300 and 1000 base pairs.

41. The kit of claim 39 wherein the restriction fragment is between 400 and 800 base pairs.

42. The kit of claim 39 wherein the plurality of barcode plasmids comprises at least 10 different plasmids and wherein the restriction enzyme is selected from the group consisting of Sty1, Nsp I, XbaI, HindIII and EcoRI.
